# EUROPÄISCHE PATENTSCHRIFT

(11) **EP 4 161 607 B1**
(45) Veröffentlichungstag und Bekanntmachung des Hinweises auf die Patenterteilung: **22.04.2026**
(21) Anmeldenummer: 21731419.4
(22) Anmeldetag: 02.06.2021
(51) Int. Cl.: A61M 1/36

(54) **VERFAHREN ZUR DISKONNEKTION**
DISCONNECTION METHOD
PROCÉDÉ DE DÉSOLIDARISATION

(30) Priorität: 05.06.2020 DE 102020114988
(43) Veröffentlichungstag der Anmeldung: 12.04.2023
(73) Patentinhaber: Fresenius Medical Care Deutschland GmbH, 61352 Bad Homburg (DE)
(72) Erfinder: HÄCKER, Jürgen, 61267 Neu-Anspach (DE)
(74) Vertreter: Lorenz Seidler Gossel Part. mbB
(86) Internationale Anmeldenummer: PCT/EP2021/064726
(87) Internationale Veröffentlichungsnummer: WO 2021/245110

(56) Entgegenhaltungen:
- JP-B2- 5 356 853

## Beschreibung

Die vorliegende Erfindung betrifft ein Verfahren zur Diskonnektion fluidführender Leitungsabschnitte eines medizinischen Geräts nach dem Oberbegriff des Anspruchs 1 sowie ein medizinisches Gerät, welches zur Ausführung eines erfindungsgemäßen Verfahrens ausgelegt ist nach dem Oberbegriff des Anspruchs 7.

Bei der Diskonnektion fluidführender Leitungsabschnitte medizinischer Geräte voneinander, wie beispielsweise eines maschinenseitigen Fluidiksystems von einem zur einmaligen Nutzung gedachten Fluidiksystems (Disposable) extrakorporaler Blutbehandlungsmaschinen oder Dialysemaschinen, sind hohe Hygienestandards einzuhalten, um die Patientensicherheit zu gewährleisten. Eine herkömmliche extrakorporale Blutbehandlungsmaschine ist beispielsweise aus der JP5356853B2 bekannt.

In der Praxis kann beispielsweise nach einer extrakorporalen Blutbehandlung beim Abkoppeln eines Schlauchsets von einer Blutbehandlungsmaschine im Schlauchset enthaltene Flüssigkeit in den geräteseitigen Fluidkreislauf bzw. die Hydraulik des Geräts gelangt und diese kontaminiert, was aufwändige Desinfektionsverfahren der Blutbehandlungsmaschine erforderlich macht.

Vor dem Beginn einer Behandlung, nach Abschluss eines Primingvorgangs (Füll- und Spülvorgang eines Fluidleitungssystems mit physiologischer Flüssigkeit) können in der Flüssigkeit in dem Fluidiksystem eines medizinischen Geräts Luft, beispielsweise in Form von Luftbläschen, vorliegen oder Kontaminationen enthalten sein. Wird das Fluidiksystem getrennt, um einen Teil des Fluidiksystems mit einem Patienten zu verbinden, soll möglichst wenig Luft in dem mit dem Patienten zu verbindendem Teil des Fluidiksystems verbleiben. Verbleibt Luft oder Kontaminationen in dem mit dem Patienten zu verbindendem Teil des Fluidiksystems kann diese bzw. können diese in den Blutkreislauf des Patienten gelangen.

Weiterhin ist es insbesondere im medizinischen Bereich wünschenswert, dass bei jedweder Diskonnektion fluidführender Leitungen Leckagen aus hygienischen Gründen vermieden werden.

Der vorliegenden Erfindung liegt somit die Aufgabe zugrunde, die Probleme des Stands der Technik abzumildern oder gar ganz zu beseitigen. Insbesondere liegt der vorliegenden Erfindung die Aufgab zugrunde, ein Verfahren zur hygienischeren Diskonnektion Fluidverbindungen und ein entsprechendes medizinisches Gerät zur Verfügung zu stellen.

Diese Aufgabe wird durch die Gegenstände der unabhängigen Ansprüche gelöst. Vorteilhafte Weiterbildungen der Erfindung sind Gegenstand der Unteransprüche.

Ein erfindungsgemäßes Verfahren zur Diskonnektion zweier fluidführender Leitungsabschnitte eines medizinischen Geräts, welche miteinander lösbar verbunden sind, weist die folgenden Schritte auf:
Einschließen eines Fluidvolumens in den zwei Leitungsabschnitten,
Erzeugen eines Unterdrucks in den zwei Leitungsabschnitten, und
Lösen der Verbindung der Leitungsabschnitte,
wobei durch das Erzeugen eines Unterdrucks in den zwei Leitungsabschnitten eine elastische Verformung im und/oder am ersten Leitungsabschnitt, welcher zumindest teilweise eine elastische Eigenschaft aufweist, aus einer Ausgangsposition in eine Spannungsposition erfolgt, wobei ein vom ersten Leitungsabschnitt umfasstes Fluidvolumen in der Spannungsposition kleiner ist als ein in der Ausgangsposition umfasstes Fluidvolumen, und
wobei sich bei dem Lösen der Verbindung der Leitungsabschnitte das vom ersten Leitungsabschnitt umfasste Fluidvolumen der Spannungsposition vergrößert.

Der Begriff "Fluid" umfasst Flüssigkeiten, Gase, Gemische von Flüssigkeiten und Gasen in gelöster Form (keine Grenzfläche) oder ungelöster Form (mit Grenzfläche). So kann die hier vorgestellte Lehre beispielsweise bei der Diskonnektion zweier Leitungsabschnitte genutzt werden, die vollständig mit Flüssigkeit oder vollständig mit Gas oder mit Flüssigkeit und Gas gefüllt sind, insbesondere auch mit einem Flüssigkeit gefüllten System das mit einem Gasreservoir verbunden ist oder mit einem Gas gefüllten System, das feucht ist, beispielsweise noch Tropfen aufweist.

Das Verfahren kann einen Schritt aufweisen, in dem die Leitungsabschnitte mit Flüssigkeit gefüllt werden.

Der Begriff "Fluidvolumen" hat die Bedeutung einer eingeschlossenen Menge. Durch das Anlegen des Unterdrucks wird ein Teil der eingeschlossenen Menge aus dem eingeschlossenen Volumen (im Sinne eines geometrischen Volumens) entfernt, d.h. das Fluidvolumen verkleinert. Die Reaktion kann darin bestehen, dass das geometrische Volumen verkleinert, indem sich beispielsweise ein Schlauchdurchmesser verkleinert. Dies ist vor allem bei näherungsweise nicht komprimierbaren bzw. expandierbaren Flüssigkeiten der Fall. Ist das eingeschlossene Volumen teilweise mit Gas gefüllt, so kann sich dieses insbesondere bei einem volumenstarren geometrischen Volumen expandieren, wenn ein Teil der flüssigen/gasförmigen Materials aus dem eingeschlossenen Volumen entfernt wird. Es sind auch Kombinationen einer Verkleinerung des geometrischen Volumens und eine Expansion eines Gases bei Verringerung des Fluidvolumens möglich.

"Elastisch" bzw. "elastischer" hat die Bedeutung, dass bei einer Diskonnektion der Verbindung ohne Eingreifen externer Kräfte der betrachtete Leitungsabschnitt eine Vergrößerung des von ihm umfassten Fluidvolumens erfährt. Beispielsweise kann der Leitungsabschnitt eine Kontraktion als Reaktion auf den Unterdruck (folglich kleineres geometrisches Volumen) und bei der Diskonnektion auf Grund der dem Leitungsabschnitt intrinsisch innewohnenden Rückstellkraft eine Relaxation unter Volumenvergrößerung zeigen und ein Nachströmen von Fluid bewirken. Dabei muss nicht, aber kann, der Leitungsabschnitt wieder zu dem Ausgangsvolumen zurückkehren. Bei einem Gas kann "Elastisch" bedeuten, dass bei Entfernen des Unterdrucks Gas bzw. Flüssigkeit nachströmt, so dass sich der Druck in der Gasphase mit dem Umgebungsdruck ausgleicht (näherungsweise gemäß idealem Gasgesetz). Das Gas mit geringerem Druck wird ebenfalls von dem Begriff "Spannungsposition" umfasst und das expandieren bzw. komprimieren wird von dem Begriff "Verformung" umfasst. "Elastischer" bedeutet, dass die Volumenvergrößerung in dem betrachteten Leitungsabschnitt größer ist als in einem Vergleichsleitungsabschnitt.

In anderen Worten kann somit die Relaxation eines verformten elastischen Leitungsabschnitts oder in einem Leitungsabschnitt bestehender Unterdruck genutzt werden, um beim Lösen einer Verbindung Fluid, insbesondere Gas oder Flüssigkeit, gezielt von einem Konnektionsbereich zweier Leitungsabschnitte wegzuleiten.

Beispielsweise wird durch das Erzeugen des Unterdrucks eine Verformung des ersten Leitungsabschnitts erzeugt, bei der sich die Innenwände der Leitung nach Innen in das Lumen wölben, der Strömungsquerschnitt somit verengt wird. Durch die Rückstellkraft des elastisch verformten Leitungsabschnitts, bewegen sich die Innenwände der Leitung wieder in Richtung der Ausgangsposition zurück und das vom ersten Leitungsabschnitt umfasstes Volumen, beispielsweise der Strömungsquerschnitt, wird wieder erweitert, wodurch Fluid in den zuvor verengten Bereich gezogen wird.

Durch die Einstellung bzw. Auswahl des Materials und/oder der Geometrie, beispielsweise Länge, Schlauchdurchmesser, Wandstärke, Rohrdurchmessergeometrie, Biegung, in bzw. von zumindest einem Teilbereich des ersten Leitungsabschnitts und / oder des zweiten Leitungsabschnitts und/oder Vorsehen von Gas, beispielsweise in Form eines Gasreservoirs, im ersten und/oder zweiten Leitungsabschnitt kann die elastische Eigenschaft des ersten Leitungsabschnitts und/oder des zweiten Leitungsabschnitts der zwei Leitungsabschnitte vorgegeben sein und somit Fluid gezielt aus dem Konnektionsbereich in Richtung einem oder beiden Leitungsabschnitts geführt werden, wenn die Leitungsabschnitte voneinander getrennt werden, insbesondere in Richtung des ersten Leitungsabschnitts, wenn er die elastische Eigenschaft aufweist. Die Rückstellkraft kann beispielsweise auch dadurch entstehen, dass ein Schlauch, der zusammengedrückt wird, wieder in die runde Form zurückkehrt oder eine gebogenere rohrförmiger Leitungsabschnitt, durch das Unterdruck Anlegen stärker gebogen wird und sich dann wiederaufrichtet oder ein gestreckter Schlauch wieder in eine gekürzte Länge zurückkehrt.

Der erste Leitungsabschnitt und der zweite Leitungsabschnitt weisen ein inneres Volumen zur Aufnahme des Fluids auf. Keiner der beiden Leitungsabschnitte ist ein Deckel oder eine Abdeckung. Bestimmungsgemäß sind der erste Leitungsabschnitt und der zweite Leitungsabschnitt dafür vorgesehen, dass bei ihrer Nutzung Flüssigkeit durch sie hindurch gefördert werden.

Der erste Leitungsabschnitt kann wenigstens in einem Teilbereich eine Elastizität aufweisen, so dass bei Anlegen eines Unterdrucks, zumindest der Teilbereich eine Kontraktion erfährt. Beim Öffnen der Verbindung zum zweiten Leitungsabschnitt kann auf Grund der Elastizität und der damit verbundenen Rückstellkraft der Leitungsabschnitt unter Vergrößerung des Volumens des Schlauchs Flüssigkeit von der Diskonnektionsstelle in die Richtung des relaxierenden Abschnitts des ersten Leitungsabschnitts verschoben werden.

Im Rahmen der vorliegenden Erfindung kann ein Teilbereich des ersten und / oder des zweiten Leitungsabschnitts oder auch der gesamte erste und / oder zweite Leitungsbereich eine elastische Eigenschaft aufweisen.

Das Fluid kann verstärkt in den ersten Leitungsabschnitt (relativ elastisches Material bzw. Geometrie im ersten Leitungsabschnitt und relativ steifes Material bzw. Geometrie im zweiten Leitungsabschnitt) oder in den zweiten Leitungsabschnitt (relativ elastisches Material bzw. Geometrie im zweiten Leitungsabschnitt und relativ steifes Material bzw. Geometrie im ersten Leitungsabschnitt) bewegt werden.

Der erste Leitungsabschnitt kann zumindest einen Teilbereich aufweisen, welcher relativ zu dem zweiten Leitungsabschnitt elastischer ist. Das kann bewirken, dass die Volumenverkleinerung beim Anlegen des Unterdrucks im ersten Leitungsabschnitt größer ausfällt als im zweiten Leitungsabschnitt. Bei der Diskonnektion erfährt der erste Leitungsabschnitt eine größere Volumenzunahme und mehr Flüssigkeit kann in Richtung des ersten Leitungsabschnitts als in Richtung des zweiten Leitungsabschnitts verschiebbar sein.

Der erste Leitungsabschnitt und der zweite Leitungsabschnitt können jeweils zumindest einen Teilbereich aufweisen, welcher relativ zu dem Rest des ersten Leitungsabschnitts und des zweiten Leitungsabschnitts aus einem elastischeren Material gefertigt ist, wobei der Teilbereich des ersten Leitungsabschnitts relativ zu dem Teilbereich des zweiten Leitungsabschnitts aus einem elastischeren Material gefertigt ist, sodass beim Lösen der Verbindung der Leitungsabschnitte, Fluid von einem Konnektionsbereich der Leitungsabschnitte in größerem Maße in den ersten Leitungsabschnitt als in den zweiten Leitungsabschnitt gesaugt wird.

In anderen Worten kann der erste Leitungsabschnitt und, optional, der zweite Leitungsabschnitt einen Teilbereich aufweisen, welcher relativ zu dem zweiten Leitungsabschnitts bzw. zu dem Rest des zweiten Leitungsabschnitts elastischer ist. Es kann aber auch der gesamte erste Leitungsabschnitt elastischer als der gesamte zweite Leitungsabschnitt sein.

Aus dem oben beschrieben geht hervor, dass ein längerer Schlauchabschnitt eine höhere Elastizität als ein kürzerer Schlauchabschnitt (bei ansonsten gleicher Geometrie und Material) aufweisen kann.

Der erste Leitungsabschnitt kann länger als der zweite Leitungsabschnitt sein.

Der erste Leitungsabschnitt und/oder der zweite Leitungsabschnitt können teilweise mit einem Gas gefüllt sein, beispielsweise Luft, während der Rest des ersten und zweiten Leitungsabschnitt mit einer Flüssigkeit gefüllt sein könne. Der mit Gas gefüllte Bereich, kann nicht unmittelbar in Verbindung mit einem Konnektionsbereich zwischen dem ersten Leitungsabschnitt und/oder dem zweiten Leitungsabschnitt stehen und/oder nicht beim Erzeugen des Unterdrucks aus dem ersten Leitungsabschnitt und/oder dem zweiten Leitungsabschnitt überführt werden.

Beispielsweise eine Pumpe Flüssigkeit aus dem ersten Leitungsabschnitt und/oder zweiten Leitungsabschnitt abpumpen. Diese Fluidvolumenabnahme führt dazu, dass dem Gas, das in einem Gasreservoir vorgesehen ist und in fluidischer Verbindung mit dem ersten und/oder zweiten Leitungabschnitt ein größeres Volumen zur Verfügung steht, so dass dieses expandiert und der Druck im Gas sinkt.

Wird anschließend die Verbindung zwischen dem ersten Leitungsabschnitt und dem zweite Leitungsabschnitt getrennt, zieht sich das Gas wieder zusammen (Druckausgleich) und gleichzeitig kann Flüssigkeit aus dem jeweils anderen Leitungsabschnitt und/oder Gas aus der Umgebung über die Diskonnektionsstelle in Richtung des Gases gesaugt werden.

Das Verfahren zur Diskonnektion kann den Schritt umfassen, ein Absperrelement angeordnet entlang des zweiten Leitungsabschnitts, an dessen einen Ende bereits ein weiteres Absperrelement angeordnet ist, zu schließen. Dadurch kann die Länge des zweiten Leitungsabschnitts verkürzt werden. Insbesondere kann es damit möglich sein, dass aufgrund unterschiedlicher elastischer Eigenschaft das Fluid bevorzugt in den ersten Leitungsabschnitt (zum Beispiel in ein Schlauchset oder ein anderes Disposable oder zum Beispiel in das interne Fluidiksystem eines Medizingeräts) gesaugt wird. Ein erfindungsgemäßes medizinisches Gerät kann mit einem derartigen Ventil ausgestattet sein.

Eine solche gezielt gerichtete Flüssigkeitsverschiebung kann genutzt werden, um die Gefahr zu reduzieren, dass unerwünschtes Material in der Flüssigkeit aus dem ersten Leitungsabschnitt in den zweiten Leitungsabschnitt oder aus der Umgebung, in den zweiten Leitungsabschnitt gelangt. Dadurch kann es möglich sein, dass eine Kontaminationsrisiko eines wiederzuverwendenden Fluidiksystems reduziert wird oder eine Menge Luft, die in einen mit einem Patienten zu verbindenden Schlauchsatz gelangt, reduziert wird.

Der Teilbereich mit der elastischen Eigenschaft kann beispielsweise eine Leitungsschlaufe oder ein Leitungspumpabschnitt einer Pumpe, insbesondere einer peristaltischen Pumpe, sein. Die Leitungsschlaufe oder der Leitungspumpabschnitt einer peristaltischen Pumpe wird während des Pumpvorgangs durch einen oder mehrere Aktoren, beispielsweise Rollen oder Finger, zusammengedrückt und relaxieren, wenn das Einwirken des oder der Aktoren entfernt wird. Vor allem bei Pumpen mit Rollen als Aktoren, kann die Leitungsschlaufe auch Zugkräften ausgesetzt sein, die zu einer Verkleinerung des Volumens führen kann. Die Leitungsschlaufe oder der Leitungspumpabschnitt einer solchen Pumpe bzw. auf die die Aktoren einer solchen Pumpe wirken weisen regelmäßig ein weicheres Material bzw. elastischeres Material als andere Leitungsabschnitte des ersten und/oder zweiten Leitungsabschnitt auf. Indem die Leitungsschlaufe oder der Leitungspumpabschnitt als Bereich höherer Elastizität verwendet wird, kann auf einen speziell zum Zwecke des hier offenbarten Diskonnektionsverfahrens vorgesehenen elastischeren Leitungsabschnitt verzichtet werden. Dadurch kann es möglich sein, eine kostengünstigere, materialsparende Lösung anzubieten. Zusätzlich kann der Aktor der peristaltischen Pumpe auch als Absperrelement genutzt werden. Dadurch kann es möglich sein weiter Kosten und Material zu sparen, da kein separates Bauteil zum Einschließen des Fluidvolumens erforderlich ist.

Alternativ oder zusätzlich können auch sowohl der erste Leitungsabschnitt als auch der zweite Leitungsabschnitt einen Teilbereich mit elastischer Eigenschaft aufweisen. Der Rest des jeweiligen Leitungsbereichs kann hierbei aus einem relativ steifen Material gefertigt sein.

Bei dieser Ausführungsform unterstützt die Relaxation der Teilbereiche aus elastischem Material bei der Diskonnektion der Leitungsabschnitte ein Abreißen der Fluidsäule. Fluid wird vom Konnektionsbereich jeweils in den ersten und zweiten Leitungsabschnitt bewegt, wodurch Leckagen vermieden und die Hygiene erhöht wird.

Auch in diesem Fall kann der elastische Teilbereich des einen Leitungsabschnitts elastischer sein als der elastische Teilbereich des anderen Leitungsabschnitts, sodass das Fluid bevorzugt in Richtung des elastischeren Teilbereichs bewegt wird.

Das erfindungsgemäße Verfahren kann ausgeführt werden, wenn noch kein Patient an ein das Verfahren ausführende Gerät angeschlossen ist.

Ein erfindungsgemäßes Verfahren kann somit der Vorbereitung eines medizinischen Geräts vor der Behandlung, beispielsweise beim Priming der eingesetzten Schlauchsets oder beim Konnektieren oder Ankoppeln der benötigten Leitungen, sowie der Nachbereitung eines medizinischen Geräts nach der Behandlung, beispielsweise beim Disonnektieren oder Abkoppeln der benötigten Leitungen, Verwerfen verwendeter Disposables, dienen.

Das erfindungsgemäße Verfahren wirkt somit nicht auf den Körper eines Patienten ein, sondern spielt sich seitens des Geräts sowie der daran angeschlossenen Disposables oder sonstiger fluidisch an das Gerät gekoppelten Komponenten ab.

Ein weiterer Aspekt der vorliegenden Erfindung betrifft ein medizinisches Gerät mit mindestens zwei voneinander lösbaren fluidführenden Leitungsabschnitten, mit wenigstens einem ersten und einem zweiten Absperrelement zum Einschließen eines Fluidvolumens in den zwei Leitungsabschnitten, einer Pumpe zum Erzeugen eines Unterdrucks in den zwei Leitungsabschnitten, und einer Steuerung zum Ansteuern der Pumpe, wobei ein erster Leitungsabschnitt der zwei fluidführenden Leitungsabschnitte zumindest teilweise eine elastische Eigenschaft aufweist und die Steuerung programmiert ist, in einem Diskonnektionsmodus die Pumpe zum Erzeugen des Unterdrucks zu betreiben, so dass durch das Erzeugen eines Unterdrucks in den zwei Leitungsabschnitten eine elastische Verformung im und/oder am ersten Leitungsabschnitt aus einer Ausgangsposition in eine Spannungsposition erfolgt.

Das medizinische Gerät kann dazu ausgelegt sein, ein Verfahren zur Diskonnektion auszuführen, oder dazu ausgelegt sein, das ein Verfahren zur Diskonnektion an diesem medizinischen Gerät ausgeführt werden kann, wobei das Verfahren zur Diskonnektion die Diskonnektion mindestens zwei fluidführenden Leitungsabschnitten, welche miteinander lösbar verbunden sind, umfasst. Ein erster Leitungsabschnitt der zwei Leitungsabschnitte weist zumindest teilweise eine elastische Eigenschaft auf.

Das medizinische Gerät weist wenigstens ein erstes und einen zweites Absperrelement zum Einschließen eines Fluidvolumens in den zwei Leitungsabschnitten auf, eine Pumpe zum Erzeugen eines Unterdrucks in den zwei Leitungsabschnitten, wodurch eine elastische Verformung des ersten Leitungsabschnitts aus einer Ausgangsposition in eine Spannungsposition erfolgen kann, und eine Steuerung zum Ansteuern der Pumpe wobei die Steuerung programmiert ist in einem Diskonnektionsmodus die Pumpe zum Erzeugen des Unterdrucks zu betreiben.

Das medizinische Gerät kann Mittel zum Einschließen eines Fluidvolumens in den zwei Leitungsabschnitten und Mittel zum Erzeugen eines Unterdrucks in den zwei Leitungsabschnitten, wodurch eine elastische Verformung zumindest eines Teilbereichs des ersten Leitungsabschnitts aus einer Ausgangsposition in eine Spannungsposition erfolgt, sodass bei einem Lösen der Verbindung der zwei Leitungsabschnitte zumindest der Teilbereich des ersten Leitungsabschnitts aus der Spannungsposition in die Ausgangsposition zurückkehrt, wodurch Fluid von einem Konnektionsbereich der Leitungsabschnitte in den ersten Leitungsabschnitt gesaugt wird, aufweisen.

Die Steuerung kann programmiert sein, vor Erzeugung des Unterdrucks eines der Absperrelemente zu schließen. Dadurch kann das Fluidvolumen einseitig abgeschlossen werden. Alternativ oder zusätzlich kann die Steuerung kann programmiert sein, nach Erzeugung des Unterdrucks eines der Absperrelemente zu schließen. Dadurch kann das Fluidvolumen eingeschlossen werden.

Die Steuerung kann mindestens eines, mehrere oder alle aktiven Bauteile ansteuern aus der Gruppe der Mittel zur Erzeugung des Unterdrucks und der Mittel zum Einschließen. Beispielsweise kann die Steuerung programmiert sein, eine Pumpe zu starten und damit einen Unterdruck zu erzeugen und / oder ein Ventil, eine Klemme zu schließen, und damit ein geschlossenes System zu erzeugen.

Der erste oder der zweiten Leitungsabschnitt kann Teil eines gerätseitigen Fluidiksystems des medizinischen Geräts ist.

Das medizinisches Gerät kann ferner eine Fluidquelle, insbesondere einer physiologischen Flüssigkeitsquelle, fluidisch verbunden mit dem zweiten Leitungsabschnitt , optional einen Sterilfilter - fluidisch angeordnet zwischen und fluidisch verbunden mit der Fluidquelle und dem zweiten Leitungsabschnitt -, und einen medizingeräteseitigen Konnektor an einem Ende des zweiten Leitungsabschnitts zum Verbinden mit einem Ende des ersten Leitungsabschnitts aufweisen.

In dieser Ausführungsform kann das medizinische Gerät eingerichtet sein, beispielsweise mittels eine Pumpe, die physiologische Flüssigkeit in einem Primingverfahren und/oder während der Behandlung über den Sterilfilter, in dem Kontaminationen zurückgehalten werden können, über den zweiten Leitungsabschnitt in den ersten Leitungsabschnitt zuzuführen, wobei der erste Leitungsabschnitt Teil eines Schlauchsystems bilden kann und mit einem während der Behandlung mit Blut gefüllten Schlauchsystem verbunden sein kann. Nach der Behandlung kann der erste Leitungsabschnitt mit dem erfindungsgemäßen Verfahren bzw. in dem Diskonnektionsmodus von dem zweiten Leitungssystem gelöst werden und beispielsweise verworfen werden. Der zweite Leitungsabschnitt kann in dem Medizinischen gerät verbleiben und bei einer nachfolgenden Behandlung wiederverwendet werden.

Das medizinische Gerät kann eine Abflussleitung aufweisen, wobei der erste Leitungsabschnitt mit der Abflussleitung fluidisch verbunden sein kann oder einen Teil der Abflussleitung bilden. Ferner kann das medizinische Gerät einen medizingeräteseitigen Konnektor an einem Ende des ersten Leitungsabschnitts zum Verbinden mit einem Ende des zweiten Leitungsabschnitt.

In dieser Ausführungsform kann der zweite Leitungsabschnitt Teil eines während der einer Blutbehandlung Blut führenden Leitung sein. Während eines Primingverfahrens kann der zweite Leitungsabschnitt mit dem ersten Leitungsabschnitt mittels des medizinigeräteseitigen Konnektor lösbar verbunden sein und Flüssigkeit, mit der der zweite Leitungsabschnitt kann in einem Spülschritt des Primingverfahrens aus dem zweiten Leitungsabschnitt in den ersten Leitungsabschnitt und weiter in die Abflussleitung überführt werden. Zum Zwecke der Behandlung kann der zweite Leitungsabschnitt von dem Konnektor bzw. dem ersten Leitungsabschnitt gelöst werden und mit dem Patienten verbunden werden.

Die Pumpe bzw. die Mittel zur Erzeugung des Unterdrucks kann eine oder mehrere Pumpen aus der Gruppe einer peristaltischen Pumpe, einer Membranpumpe, einer Zentrifugalpumpe, einer Impellerpumpe und einer Zahnradpumpe sein.

Die Pumpe bzw. die Mittel zur Erzeugung des Unterdrucks kann eine oder mehrere Pumpen aus der Gruppe einer Ultrafiltrationspumpe, einer Blutpumpe, einer Substituatpumpe sein.

Die Pumpe kann eine peristaltische Pumpe sein und wenigstens ein Aktor der peristaltischen Pumpe kann Teil des ersten oder zweiten Absperrelements sein.

Das oder die Absperrelement bzw. das oder die Mittel zum Einschließen können jeweils eine oder mehrere Pumpen, Ventilen, Rückschlagventilen oder Klemmen oder einer Kombination dieser aufweisen.

Die Absperrelemente können manuell zu bedienende Bauteile sein oder durch die Steuerung ansteuerbar sein. Es kann auch ein erstes Absperrelement manuell und ein zweites Absperrelement durch die Steuerung betätigbar sein. Es können auch mehrere Absperrelemente vorgesehen sein.

Der erste und/oder der zweite Leitungsabschnitt kann verzweigt sein. An jedem Ende des der ersten und/oder der zweiten Leitungsabschnitt, außer an lösbaren Verbindungsenden des ersten und zweiten Leitungsabschnitt, kann ein je ein Absperrelement vorgesehen sein. kann verzweigt sein.

Bei den Ausführungsformen mit ansteuerbaren Absperrelementen, kann die Steuerung programmiert sein, die ansteuerbaren Absperrelemente anzusteuern, um das Fluidvolumen einzuschließen. Beispielsweise kann die Steuerung programmiert sein, die Pumpe in eine Richtung zu betreiben. Ferner kann die Steuerung programmiert sein, in einem ersten Schritt alle stromauf angeordneten Absperrelemente zu schließen, in einem nachfolgenden Schritt die Pumpe zu betreiben und in einem nachfolgenden Schritt alle stromab angeordneten Absperrelemente zu schließen. Alternativ können auch im ersten Schritt eine oder mehrere oder alle stromab gelegenen ansteuerbaren Absperrelemente geschlossen werden solange eine offene Verbindung nach zum Abführen von Fluid existiert. Dabei kann es sich beispielsweise um ein Rückschlagventil oder eine manuell zu schließende Klemme handeln.

Das medinische Gerät kann eine Benutzerschnittstelle zum Eingeben einer Anweisung durch einen Nutzer aufweisen und die Steuerung kann programmiert sein, den Diskonnektionsmodus zu aktivieren als Reaktion auf eine Eingabe der Anweisung an der Benutzerschnittstelle.

Bei der Aktivierung des Diskonnektionsmodus kann ein spezieller Modus im Programmcode der Steuerung aktiviert werde. Bei der Aktivierung des Diskonnektionsmodus kann eine spezielle Sequenz eines Programmcodes ablaufen, mit denen das medizinische Gerät das Diskonnektionsverfahren durchführt. Der Diskonnektionsmodus kann auch in einen anderen Modus integriert sein.

Beispielsweise kann ein Primingmodus im Programmcode hinterlegt sein und der Diskonnektionsmodus kann einen Schritt, beispielsweise den letzten Schritt des Primingmodus darstellen.

Die Benutzerschnittstelle kann eine Anzeige, ein Bildschirm, ein Touchscreen, eine Tastatur, ein Bedienknopf, ein Microphon zur Aufnahme eines Sprachsignals, eine Kamera zu Erkennung einer Geste des Nutzers.

Die Steuerung kann programmiert sein, eine Mehrzahl von Modi zu aktivieren und einen Wechsel aus einem der Modi in den Diskonnektionsmodus automatisch durchzuführen.

Beispielsweise kann die Steuerung in einem sogenannten Reinfusionsmodus mit einem Sensor des Medizinischen Gerätes, beispielsweise einem optischen Sensor, verbunden sein, der erkennt, dass kein Blut mehr im Schlauchsatz vorliegt. Dieses Signal kann die Steuerung in seinem Programmcode derart verarbeiten, dass ein weiterer Modus gestartet werden kann. Bei diesem Modus kann es sich um den Diskonnektionsmodus handeln. Alternativ oder zusätzlich kann eine zusätzlichen Modus im Programmcode der Steuerung vorgesehen sein, der nach Abschluss des Reinfusionsmodus ein Leeren des Schlauchsatzes und/oder eines Dialysators und/oder eines maschinenseitigen Fluidsystems, und die Steuerung kann programmiert sein, nach einem vorgegebenen Zeitintervall oder nachdem ein vorgegebenes Fluidvolumen transportiert wurde oder nachdem Luft oder eine Grenzfläche zwischen Blut und einer Reinfusionsflüssigkeit mit anderen optischen Eigenschaften als Blut im zu leerenden Bereich mittels eines Sensors erkannt wurde oder nachdem ein vorgegebener Druck erkannt wird, in den Diskonnektionsmodus zu schalten.

Alternativ oder zusätzlich kann ein Modus ("Primingmodus") im Programmcode der Steuerung vorgesehen sein, der - vor Beginn der Behandlung - das Schlauchset oder die Kassette mit Priming Flüssigkeit füllt und/oder das Schlauchset bzw. die Kassette mit Flüssigkeit spült, und die Steuerung kann programmiert sein, nach Abschluss des Primingsmodus in den Diskonnektionsmodus zu schalten. Die Steuerung kann programmiert sein, während des Primingmodus eine Pumpe zu betreiben und damit Flüssigkeit aus einer Flüssigkeitsquelle in Richtung des Schlauchsets bzw. der Kassette zu verschieben. Die Steuerung kann programmiert sein, ein Ende des Primings festzustellen, indem beispielsweise eine vorgegebene Zeit abgelaufen ist, ein vorgegebene Flüssigkeitsvolumen in das Schlauchset bzw. die Kassette verschoben wurde, ein Luftdetektor bzw. Flüssigkeitsensor anzeigt, dass keine Luft mehr bzw. überwiegend nur Flüssigkeit vorliegt.

Die Steuerung kann auch derart programmiert sein, dass zunächst automatisch in den Diskonnektionsmodus gewechselt wird, die Pumpe jedoch nur nach Eingabe über die Benutzerschnittstelle gestartet wird.

Die Steuerung kann ferner programmiert sein, eine Eingabe über die Benutzerschnittstelle zum Starten der Pumpe, insbesondere nur dann, zuzulassen, wenn der Diskonnektionsmodus aktiviert ist. Mit anderen Worten kann der Programmcode der Steuerung vorsehen, dass es Modi oder Phasen gibt, die nicht der Diskonnektionsmodus sind, in denen ein Starten der Pumpe nicht erfolgt trotz entsprechender Eingabe über die Benutzerschnittstelle.

Mit einer solchen Begrenzung des Zulassens des Starten des Diskonnektionsverfahrens kann es möglich sein, zu verhindern, dass beispielsweise während er Behandlung ein solches Diskonnektionsverfahren gestartet werden kann.

Wie oben offenbart kann das medizinische Gerät weder den ersten noch den zweiten Leitungsabschnitt aufweisen, sondern lediglich mit diesen verbunden werden zum Zwecke eine Behandlung.

Wie oben offenbart kann das medizinische Gerät einen der beiden Leitungsabschnitt aufweisen, und lediglich mit dem zweiten der beiden Leitungsabschnitte zum Zwecke eine Behandlung verbunden werden.

In einer weiteren Ausführungsform weist das medizinische Gerät beide, den ersten und den zweiten, Leitungsabschnitt auf. Der erste Leitungsabschnitt kann zumindest teilweise eine elastische Eigenschaft aufweisen, wobei der erste Leitungsabschnitt durch die elastische Eigenschaft elastisch verformbar aus einer Ausgangsposition in eine Spannungsposition ist, wobei ein vom ersten Leitungsabschnitt umfasstes Volumen in der Spannungsposition kleiner ist als ein in der Ausgangsposition umfasstes Volumen.

Optional kann einer der beiden Leitungsabschnitte Teil eines Disposables sein, insbesondere eines im Rahmen einer Blutbehandlung zum Einsatz kommenden Schlauchsets oder Kassettensystems

Optional können beide Leitungsabschnitte Teil eines oder mehrerer Disposables sind, insbesondere eines im Rahmen einer Blutbehandlung zum Einsatz kommenden Schlauchsets oder Kassettensystems oder eine als Disposable gestalteten Hydraulik (maschinenseitige Fluidiksystem, das während der Behandlung nicht von Blut durchströmt wird) zur Versorgung eines Dialysators mit Dialysierflüssigkeit.

Der erste Leitungsabschnitt kann wenigstens einen Teilbereich aufweisen, welcher relativ zu dem zweiten Leitungsabschnitts elastischer ist.

Gemäß einer Ausführungsform weisen der erste Leitungsabschnitt und der zweite Leitungsabschnitt zumindest einen Teilbereich auf, welcher relativ zu dem Rest des ersten Leitungsabschnitts und des zweiten Leitungsabschnitts aus einem elastischeren Material gefertigt ist, wobei der Teilbereich des ersten Leitungsabschnitts relativ zu dem Teilbereich des zweiten Leitungsabschnitts aus einem elastischeren Material gefertigt ist, sodass beim Lösen der Verbindung der Leitungsabschnitte, Fluid von einem Konnektionsbereich der Leitungsabschnitte in größerem Maße in den ersten Leitungsabschnitt als in den zweiten Leitungsabschnitt gesaugt wird, oder umgekehrt.

Die Steuerung kann beispielsweise ein Computersystem aufweisen und können in Form von digitalen Schaltkreisen, Computerhardware, Firmware, Software oder in beliebigen Kombinationen davon implementiert werden. Die Erfindung kann weiterhin in Form eines Computerprogrammprodukts, z.B. eines Computerprogramms auf einem physischen Informationsträger (z.B. maschinenlesbares Speichermedium) implementiert werden. Die Steuerung kann einen allgemeinen Prozessor, einen Digitalen Signalprozessor (DSP) zur kontinuierlichen Bearbeitung digitaler Signale, einen Mikroprozessor, eine anwendungsspezifische integrierte Schaltung (ASIC), einen aus Logikelementen bestehenden integrierten Schaltkreis (FPGA) oder andere integrierte Schaltkreise (IC) oder Hardware -Komponenten aufweisen, um die einzelnen Verfahrensschritte auszuführen. Auf den Hardware-Komponenten kann zur Durchführung der Verfahrensschritte ein Datenverarbeitungsprogramm (Software) laufen. Es ist auch eine Mehrzahl oder Kombination der verschiedenen Komponenten möglich, um deren Betrieb zu steuern.

Die Steuerung kann ferner einen Speicher aufweisen, indem der Programmcode abgelegt ist, beispielsweise einen Read-Only Memory (ROM) oder Random Access Memory (RAM) oder beide, magnetische, magneto-optische, optische oder solid state (SSD) Speichermedien, nicht-flüchtigen Speicherelementen wie Halbleiterspeicherelemente (z.B. EPROM, EEPROM), Flash-Memory Vorrichtungen, magnetische oder magneto-optische Speichermedien, CD-ROM, DVD-ROM oder Blue-Ray Disks. Der Speicher kann auch bei Bedarf (on demand) bereitgestellt werden oder über das Internet erreichbar sein (z.B. Cloud Computing). Geeignete Datenträger zum Abspeichern von Programminstruktionen und Daten umfassen alle Arten von nicht-flüchtigen Speicherelementen wie Halbleiterspeicherelemente (z.B. EPROM, EEPROM), Flash-Memory Vorrichtungen, magnetische oder magneto-optische Speichermedien, CD-ROM, DVD-ROM oder Blue-Ray Disks. Die Prozessor- und Speicherelemente können ergänzt werden durch spezielle Logikbausteine, oder auch Teil dieser sein.

Weitere Merkmale und Effekte der vorliegenden Erfindung ergeben sich aus der nachfolgenden Beschreibung ausgewählter Ausführungsformen der Erfindung unter Bezugnahme auf die beigefügten Figuren, in denen gleiche oder ähnliche Bauteile mit denselben Bezugszeichen bezeichnet sind. Die im folgenden beschriebenen Merkmale könne in weiter oben beschriebenen Ausführungsformen verwirklicht sein. Diese weiter oben beschriebenen Ausführungsformen werden im Folgenden nicht alle noch einmal angeführt. Hierbei zeigen:
Fig. 1a zwei Leitungsabschnitte, welche im Rahmen der des Verfahrens zur Diskonnektion zweier fluidführenden Leitungsabschnitte zum Einsatz kommen, und das medizinische Gerät;
Fig. 1b einen ersten Leitungsabschnitt der zwei fluidführenden Leitungsabschnitte in einer Spannungsposition am Beispiel einer mechanischen Verformung;
Fig. 1c den ersten Leitungsabschnitt der Fig. 1b in einer Ausgangsposition / Entspannungsposition;
Fig. 2 eine Ausführungsform mit einer Pumpe als Absperrelement;
Fig. 3 eine Ausführungsform mit einem Rückschlagventil als Absperrelement;
Fig. 4 eine Ausführungsform mit einer Hydraulik als Teil des ersten Leitungsabschnitts;
Fig. 5a eine Ausführungsform mit einer außerhalb angeordneten Pumpe;
Fig. 5b eine Ausführungsform mit einem verzweigten Leitungsabschnitt;
Fig. 6a eine Ausführungsform mit einem T bzw. Y Stück zur Konnektion zweier Leitungen;
Fig. 6b eine Ausführungsform, bei der das T bzw. Y Stück an einem Ende eines Leitungsabschnitts angeordnet ist;
Fig. 7a eine Ausführungsform eines medizinischen Geräts;
Fig. 7b eine Ausführungsform des medizinischen Geräts in einer anderen Konfiguration; und
Fig. 8a und 8b Ausführungsformen der Benutzerschnittstelle.

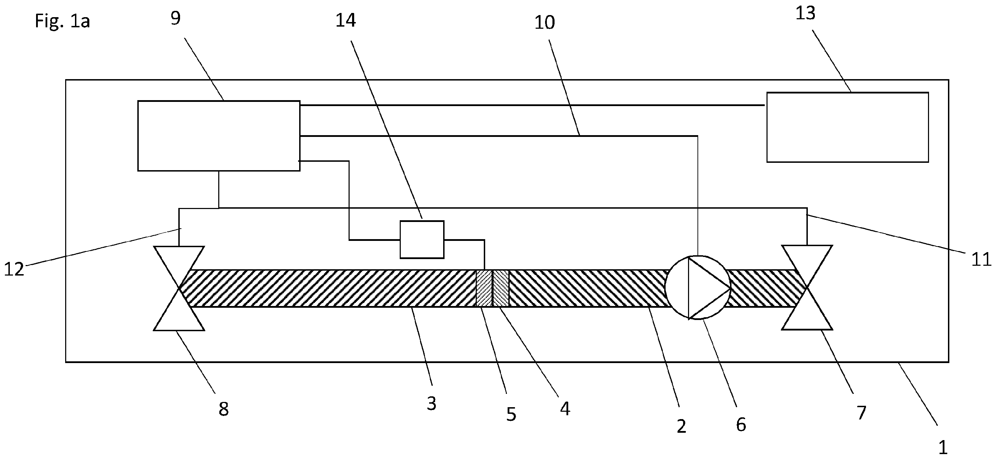

Eine Ausführungsform des medizinischen Geräts 1 , schematisch dargestellt in Fig. 1a, weist bei Anwendung des erfindungsgemäßen Verfahrens zwei fluidisch verbundene Leitungsabschnitte 2, 3 auf, welche miteinander über zwei optionale Konnektorelemente 4, 5 der zwei Leitungsabschnitte fluidisch verbunden sein können. Ferner weist das medizinische Gerät 1 eine Pumpe 6 auf. An jeweils einem Ende weisen der erste und der zweite Leitungsabschnitt je ein Absperrelement 7, 8 auf. Der Leitungsabschnitt 2 weist zumindest teilweise eine elastische Eigenschaft auf, insbesondere einen elastisch verformbaren Teilbereich. Die elastische Eigenschaft bzw. der verformbare Teilbereich kann zwischen der Konnektionsstelle zum zweiten Leitungsabschnitt 3 bzw. zwischen dem Konnektorelement 4 und dem Absperrelement 7 verkörpert bzw. angeordnet sein. Mittels der Pumpe 6 kann ein Unterdruck in dem ersten Leitungsabschnitt 2 oder in dem ersten Leitungsabschnitt 2 und dem zweiten Leitungsabschnitt 3 erzeugt werden. Durch den Unterdruck kann im und/oder am ersten Leitungsabschnitt 2 eine Verformung aus einer Ausgangsposition in eine Spannungsposition erfolgen, wobei ein vom ersten Leitungsabschnitt 2 umfasstes Fluidvolumen in der Spannungsposition kleiner ist als ein in der Ausgangsposition umfasstes Fluidvolumen.

Die Absperrelemente 7, 8 können geschlossen werden und dadurch ein Fluidvolumen in den zwei Leitungsabschnitten 2, 3 eingeschlossen werden. Das Schließen der Absperrelemente7, 8 kann nacheinander oder gleichzeitig erfolgen, insbesondere kann zunächst ein erstes Absperrelement geschlossen werden, dann der Unterdruck erzeugt werden, und anschließend das zweite Absperrelement geschlossen werden.

Anschließend kann ein Lösen der Verbindung der Leitungsabschnitte 2, 3 erfolgen, wobei sich das vom ersten Leitungsabschnitt 2 umfasste Fluidvolumen im Vergleich zum Fluidvolumen in der Spannungsposition vergrößert.

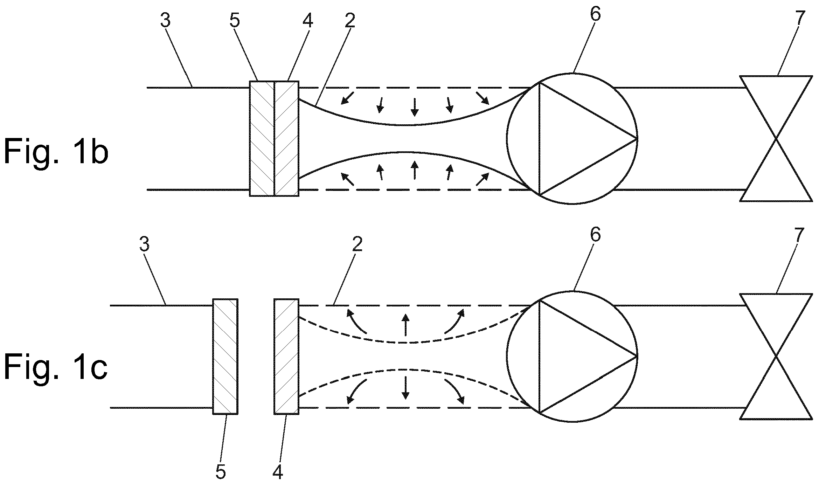

Anhand der Fig. 1b und 1c wird das Verfahren anhand einer elastischen mechanischen Verformung erläutert. Wie beschrieben, ist das lediglich eine Möglichkeit von mehreren Möglichkeiten, wie die elastische Eigenschaft verwirklicht werden kann.

Ist das Absperrelement 8 am Ende des zweiten Leitungsabschnitts geschlossen und pumpt die Pumpe 6 Fluid bzw. Flüssigkeit aus dem ersten Leitungsabschnitt 2 in Richtung des zweiten Absperrelements 7 (offen) wird ein Unterdruck zumindest in einem Teil des ersten Leitungsabschnitts 2 erzeugt. Dabei kann sich das Volumen des ersten Leitungsabschnitt 2 verformen, beispielsweise nach innen (siehe die Pfeile in Fig. 1b) zusammenziehen, sodass der das Volumen des ersten Leitungsabschnitt 2 verringert wird. Werde in diesem Zustand der erste und der zweite Leitungsabschnitt 2, 3 eingeschlossen, beispielsweise durch Schließen des Absperrelements 7 am Ende des ersten Leitungsabschnitts 2, verbleibt das System mit dem verringerten Volumen.

Mit anderen Worten verlässt die Wandung des Leitungsabschnitts 2 somit die, in Fig. 1a schematisch gestrichelt eingezeichnete, Ausgangsposition und nimmt eine Spannungsposition ein, welche durch eine durchgezogene Linie in Fig. 1b widergegeben ist.

Werden die zwei Leitungsabschnitte 2, 3 voneinander gelöst, so kommt es auf Grund der elastischen Eigenschaft zum Relaxieren des elastisch verformbaren Teilbereichs und der erste Leitungsabschnitt 2 kehrt aus der Spannungsposition in die Richtung der Ausgangsposition zurück. Wie in Fig. 1c gezeigt, bewegt sich die Wandung des Leitungsabschnitts 2 beispielsweise dadurch wieder aus der Spannungsposition (gestrichelte Linie) in Richtung der Ausgangsposition oder in die Ausgangsposition (durchgezogene Linie). Dabei kann Fluid vom Konnektionsbereich oder anders ausgedrückt vom Diskonnektionsbereich, beispielsweise im Bereich des Konnektorelements 4, in dem in den Figuren 1a-1c gezeigten Beispiel nach rechts, in den zweiten Leitungsabschnitt 2 gesaugt werden.

Vor dem Trennen des ersten von dem zweiten Leitungsabschnitt kann sich im Konnektionsbereich Flüssigkeit bzw. eine Flüssigkeitssäule befinden. Ohne die Saugwirkung durch die Relaxation könnte die Flüssigkeitssäule einfach zerrissen werden und die Flüssigkeit würde zumindest der Schwerkraft folgend nach unten außen dem Konnektionsbereich auslaufen.

Beim Trennen des ersten von dem zweiten Leitungsabschnitt 2, 3 im hier beschriebenen Fall kann auf Grund der Relaxation im ersten Leitungsabschnitt 2 die Luft von außen nach innen im Diskonnektionsbereich gesaugt werden auch die Fluidsäule zumindest teilweise ebenfalls der Saugwirkung folgen. Dadurch kann insgesamt mehr Flüssigkeit im ersten Leitungabschnitt 2 beim Trennen verbleiben und es kann auch eine gerichtete Strömung der Flüssigkeit in den ersten Leitungsabschnitt 2 erreichbar sein.

Somit kann das Risiko einer Kontamination des zweiten Leitungsabschnitts 2 und/oder einer Leckage (Austritt von Flüssigkeit nach außen) reduziert werden.

Das medizinische Gerät 1 kann eine Steuerung 9 aufweisen. Die Steuerung 9 kann programmiert sein in einem Diskonnektionsmodus die Pumpe zum Erzeugen des Unterdrucks zu betreiben. Optional kann die Steuerung 9 auch programmiert sein, wenigstens eines oder beide Absperrelemente 7, 8 anzusteuern. Dazu kann die Steuerung über Signalleitungen 10, 11, 12 des medizinischen Geräts 1 mit den jeweiligen anzusteuernden Bauteilen (beispielsweise Pumpe 6, Absperrelemente 7, 8) verbunden sein. Die Steuerung 9 kann beispielsweise programmiert sein, die Pumpe 6 zu starten und/oder zu stoppen.

Beispielsweise kann die Steuerung programmiert sein, die Pumpe 6 bei geschlossenem ersten Ventil 7 oder geschlossenem Ventil 8, zu starten und das jeweils andere Ventil 7, 8 zu schließen, so dass ein eingeschlossenes Volumen, in dem ein Unterdruck anliegt bzw. sich der verformbare Teilbereich verformt hat, entsteht.

Das medizinische Gerät 1 kann eine Benutzerschnittstelle 13 aufweisen. Benutzerschnittstelle 13 kann eingerichtet sein zum Eingeben einer Anweisung durch einen Nutzer und die Steuerung 9 kann programmiert sein, den Diskonnektionsmodus zu aktivieren als Reaktion auf eine Eingabe der Anweisung an der Benutzerschnittstelle 13.

Die Leitungsabschnitte 2, 3 sind nicht beide zwingend Teil des medizinischen Geräts 1 sondern einer oder beide der Leitungsabschnitte 2,3 können erst bei Verwendung des medizinischen Geräts 1 mit der Pumpe 6 und den Absperrelementen 7, 8 verbunden werden.

Das medizinische Gerät 1 kann den ersten Leitungsabschnitt 2 und / oder den zweiten Leitungsabschnitt 3 aufweisen. Der erste Leitungsabschnitt 2 und / oder der zweiten Leitungsabschnitt 3 können Teil eines gerätseitigen Fluidiksystems des medizinischen Geräts 1 sein.

Der erste Leitungsabschnitt 2 und / oder der zweiten Leitungsabschnitt 3 können Teil eines Disposable sein.

Die Pumpe 6 kann entlang des erste Leitungsabschnitt 2 und / oder der zweiten Leitungsabschnitt 3 oder einer Stelle eines Fluidiksystem außerhalb des zwei Leitungsabschnitt 2, 3, die in fluidischer Verbindung mit den zwei Leitungsabschnitten 2, 3 stehen, angeordnet sein. Beispielsweise kann die Pumpe 6 auf der jenseits der Konnektoren 3, 4 liegenden Seite der Absperrelemente 7,8 angeordnet sein. Es muss mit der Pumpe 6 lediglich möglich sein, Flüssigkeit aus dem elastisch verformbaren Teilbereich zu entfernen.

Das medizinische Gerät 1 kann eine automatische Diskonnektionsvorrichtung 14 aufweisen. Diese automatische Diskonnektionsvorrichtung 14 kann beispielsweise einen Motor aufweisen, der den ersten und/oder den zweiten Leitungsabschnitt 2, 3 verschiebt und dadurch die Verbindung zwischen dem ersten und den zweiten Leitungsabschnitt 2, 3 löst. Die Steuerung 9 kann dazu programmiert sein, die automatische Diskonnektionsvorrichtung 14 anzusteuern. Das kann es ermöglichen, dass das hygienische Diskonnektionsverfahren vollautomatisch, d.h. ohne Eingriff eines Menschen erfolgen kann.

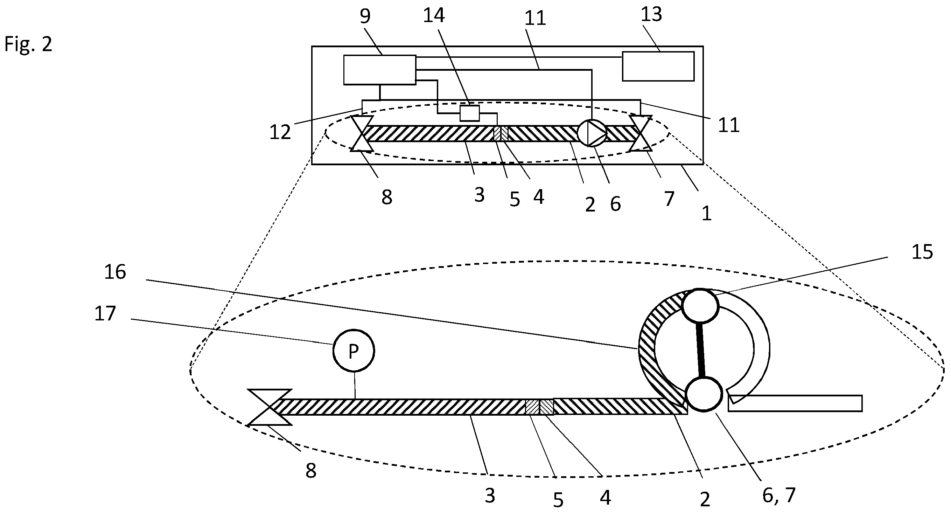

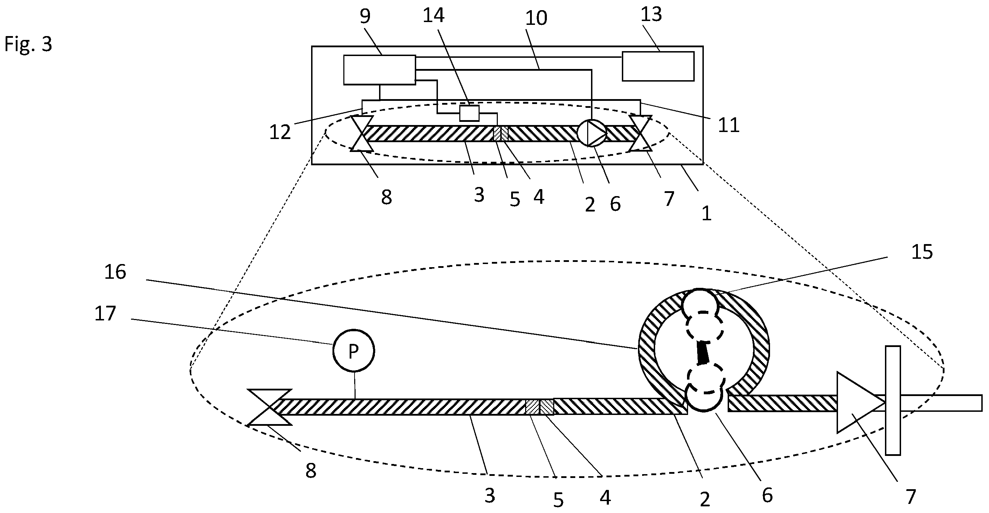

In Fig. 2 ist eine Ausführungsform gezeigt, bei welcher eine Pumpe 6 als Absperrelement 7 bzw. als Mittel zum Einschließen eines Fluidvolumens dient. Die Darstellung in Figur 2 ist keine Vergrößerung, sondern soll den Vergleich der Komponenten der jeweiligen Ausführungsformen des medizinischen Geräts 1 , wie beispielsweise in Fig. 1 schematisch dargestellt, erleichtern. Die Steuerung 9, die Benutzerschnittstelle 13, die Signalleitungen 10, 11, 12, die automatische Diskonnektionseinrichtung 14 können alle oder teilweise auch in dieser Ausführungsform vorhanden sein und bezüglich dieser wird auf die Beschreibung zu der Figuren 1a bis 1c verwiesen. Ausgestaltungen der Steuerung 9, der Benutzerschnittstelle 13, der Signalleitungen 10, 11, 12, der automatische Diskonnektionseinrichtung 14, die im Folgenden beschrieben werden, können soweit technisch sinnvoll auch in dem medizinischen Gerät 1, wie im Zusammenhang mit Figuren 1a bis 1c beschrieben, vorliegen. Dasselbe gilt für die Figuren 3 bis 8a.

Die Pumpe 6 kann eine peristaltische Pumpe sein und ein Aktor 15 kann mit dem ersten Schlauchabschnitt 2 im Bereich einer Leitungsschlaufe 16 in Eingriff stehen. Dadurch kann das abgeschlossenes Fluidvolumen gebildet werden.

Die Pumpe 6 ist in diesem Beispiel eine Rollenpumpe (eine Ausführungsform einer peristaltischen Pumpe) und die Leitungsschlaufe 16 ist in die Pumpe 6 eingelegt.

Vor der Diskonnektion der Leitungsabschnitte 1 und 2 werden die Rollen der Pumpe 6 in eine vorgegebene Diskonnektionsposition verfahren, sodass wenigstens ein Teil der Leitungsschlaufe 16 innerhalb des zweiten Abschnitts (zwischen der Konnektionsstelle und dem fluidischen Absperrpunkt der Pumpe 6) liegt, und verbleiben in dieser schließenden Konfiguration bei der Diskonnektion. In diesem Beispiel dient die Pumpe 6 als Absperrelement seitens des ersten Leitungsabschnitts 2. Seitens des Geräts dient das Ventil 8 als Absperrelement. Die Pumpe 6 dient auch zur Erzeugung des Unterdrucks in den miteinander verbundenen Leitungsabschnitten 1 und 2.

Die Rollen der Pumpe 6 führen zur Erzeugung von Unterdruck mindestens eine Drehung um einen vorgegebenen Winkel durch. Wenn die Stellung der Rollen beim Starten des Vorgangs, so ist, dass der Winkel nicht mehr groß genug ist, kann zusätzlich um eine volle Umdrehung oder halbe Umdrehung bei zwei Rollen gedreht werden. Die Drehung kann auch solange erfolgen, bis ein vorgegebener Unterdruck erreicht ist.

Ein Drucksensor 17 kann in dem medizinischen Gerät 1 vorgesehen sein, der den Druck im eingeschlossenen Volumen misst und gegebenenfalls im Zusammenspiel mit der Benutzerschnittstelle 13 den Druck anzeigt oder anzeigt, wenn der Unterdruck ausreicht, und / oder der Steuerung 9 anzeigt, dass die Pumpe 6 weiterzudrehen ist.

Die Steuerung kann programmiert sein, die Pumpe entsprechend anzusteuern.

Beim Lösen des ersten vom zweiten Leitungsabschnitt 1, 2, bewegt sich der zweite Leitungsabschnitt 2 bzw. dessen Teilbereichs 16 in die Ausgangsposition zurück und Fluid wird in den zweiten Leitungsabschnitt 2 bzw. dessen Teilbereich 16 gesaugt.

Wie in Fig. 3 gezeigt, kann eine Pumpe 6 in Kombination mit einem Absperrorgan 8 in Form eines Rückschlagventils zum Einsatz kommen. Das fluidische Abschließen des ersten Leitungsabschnitts 2 erfolgt somit durch das Rückschlagventil, dessen Durchlassrichtung weg vom Konnektor bzw. vom zweiten Leitungsabschnitt 3 verläuft.

Mittels der Pumpe 6 wird Fluid, insbesondere Flüssigkeit, durch das Rückschlagventil 8 hindurch gefördert, Wodurch der erste Leitungsabschnitt 2, insbesondere dessen Teilbereich 16 in eine Spannungsposition gebracht wird. In dieser Ausführungsform kann das eingeschlossene Volumen bereits vor einem Starten der Pumpe vorliegen. Bei betreiben der Pumpe wird Fluid aus dem Bereich des ersten und/oder zweiten Leitungsabschnitt 2, 3 entfernt.

Verschiedene Pumpentypen sind im Rahmen beispielsweise dieser Ausführungsform einsetzbar, da in diesem Beispiel okkludierende Eigenschaften nicht für das Einschließen des Fluidvolumens erforderlich sind, z.B. kann eine peristaltische Pumpe 6 mit zurückziehbaren Aktoren, Zahnradpumpe, Impellerpumpe, Zentrifugalpumpe oder eine Membranpumpe eingesetzt werden.

Das Rückschlagventil kann aber auch in der im Zusammenhang mit Fig. 2 beschriebenen Ausführungsform vorliegen. Das kann bewirken, dass ein zusätzlicher Schutz bereitgestellt wird, dass keine Flüssigkeit in den Bereich der Verbindung zwischen den beiden Leitungsabschnitte gelangt, da sowohl die Sperrwirkung des Rückschlagventils als auch die Absperrwirkung des Aktors überwunden werden muss. Eine solche zusätzliche Maßnahme kann insbesondere bei peristaltischen Pumpen sinnvoll sein, da konstruktiv bedingt, die Rollen regelmäßig federnd gelagert sind und bei zu großen Kräften abheben und damit ihre okkludierende Funktion verlieren können.

Die Höhe des erzeugten Unterdrucks kann durch vorgegebene Anzahl an Pumpendrehungen / Betätigungen der Finger eine Fingerpumpe oder mittels eines vorgegebenen Unterdrucks eingestellt werden. Diese Steuerung bzw. Überwachung kann durch die Steuerung 9 erfolgen.

Bei Lösen der ersten und zweiten Leitungsabschnitte 1 und 2, bewegt sich der zweite Leitungsabschnitt 2 bzw. dessen Teilbereich 16 in die Ausgangsposition zurück und Fluid wird in den zweiten Leitungsabschnitt 2 bzw. dessen Teilbereich 16 gesaugt.

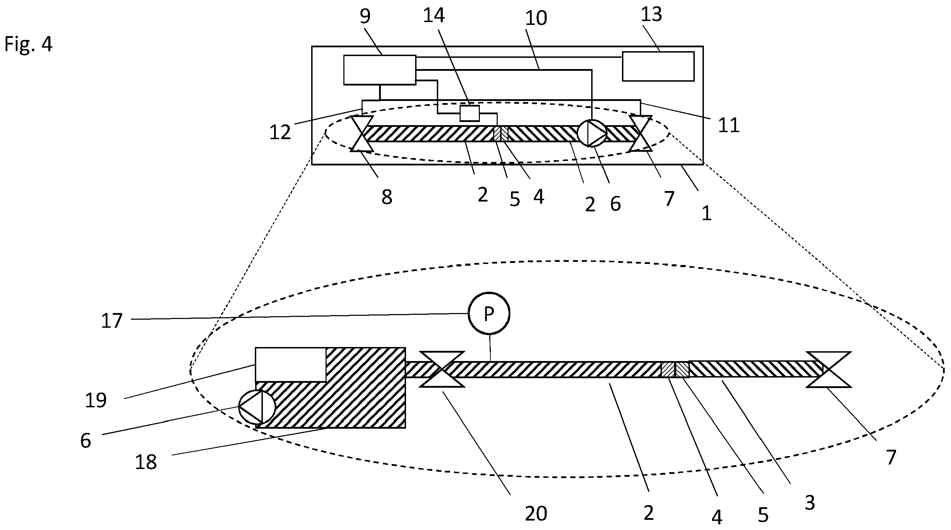

Das medizinischen Gerät 1, schematisch gezeigt in Fig. 4, kann eine geräteseitige Hydraulik 18 aufweisen, die mit dem ersten Leitungsabschnitt 2 in fluidischer Verbindung stehen kann oder einen Teil des ersten Leitungsabschnitts 2 bilden kann. Die geräteseitige Hydraulik 18 kann fluidisch abschließbar sein mittels eines oder mehrerer Absperrelemente. Die geräteseitige Hydraulik 18 kann ein Gasreservoir 20 aufweisen. Bei Betrieb des medizinischen Geräts 1 kann der erste und der zweite Leitungsabschnitt 2, 3 und die Hydraulik 18 mit Ausnahme des Gasreservoirs 19 mit Flüssigkeit gefüllt sein. Eine Pumpe 6 kann vorgesehen sein, um den Unterdruck zu erzeugen. Bei der Pumpe 6 kann es beispielsweise Pumpe handeln, die während einer Blutbehandlung eine Flüssigkeit, beispielsweise während einer Dialysebehandlung Dialysat, pumpt. Die Pumpe 6 kann eine Ultrafiltrationspumpe oder eine Bilanzierpumpe sein.

Bei einer solchen Anordnung, kann Fluid in Richtung des ersten Leitungsabschnitts 2 als Teil einer der geräteseitigen Hydraulik 18 gesaugt werden. Diese Anordnung kann verwendet werden, wenn ein mit Flüssigkeit gefülltes Schlauchsatz ,beispielsweise nach dem Primen, zumindest teilweise gebildet durch den zweiten Leitungsabschnitt 3 von dem ersten Leitungsabschnitt 1 entfernt werden soll und daraufhin mit einem Patienten verbunden werden soll. Dadurch kann möglicherweise der zweite Leitungsabschnitt 3 in einem hygienischeren Zustand gehalten werden.

Das medizinische Gerät 1 kann ein Absperrmittel 20 aufweisen. Das Absperrmittel 20 kann geöffnet werden bzw. die Steuerung 9 kann programmiert sein, es zu öffnen bevor der Unterdruck erzeugt wird. Dadurch kann das Volumen (durch das zusätzliche Volumen der Hydraulik 18), in dem der Unterdruck wirkt, vergrößert werden bzw. das Fluidvolumen der ersten Leitungsabschnitts 2 kann vergrößert werden und/oder das Gasreservoir kann Teil des ersten Leitungsabschnitts 2 werden. Dies kann eine elastischere Eigenschaft des ersten Leitungsabschnitts 2 bewirken, als wenn das Absperrelement 20 geschlossen ist. Damit kann die Wirkung der Relaxation aus der Spannungsposition vergrößerbar sein.

Die geräteseitige Hydraulik 18 kann einen Teilbereich aufweisen aus einem elastischen Material. Der Teilbereich ist durch den erzeugten Unterdruck in eine Spannungsposition bewegbar.

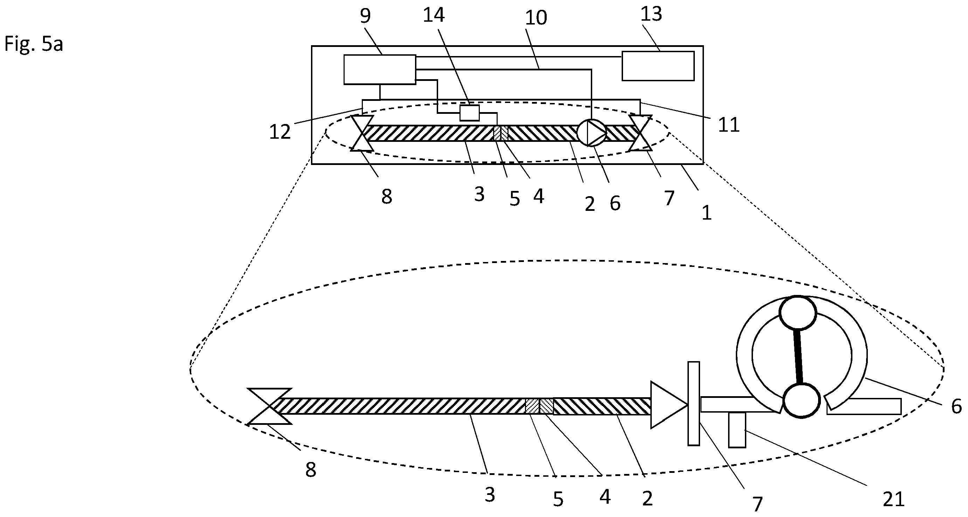

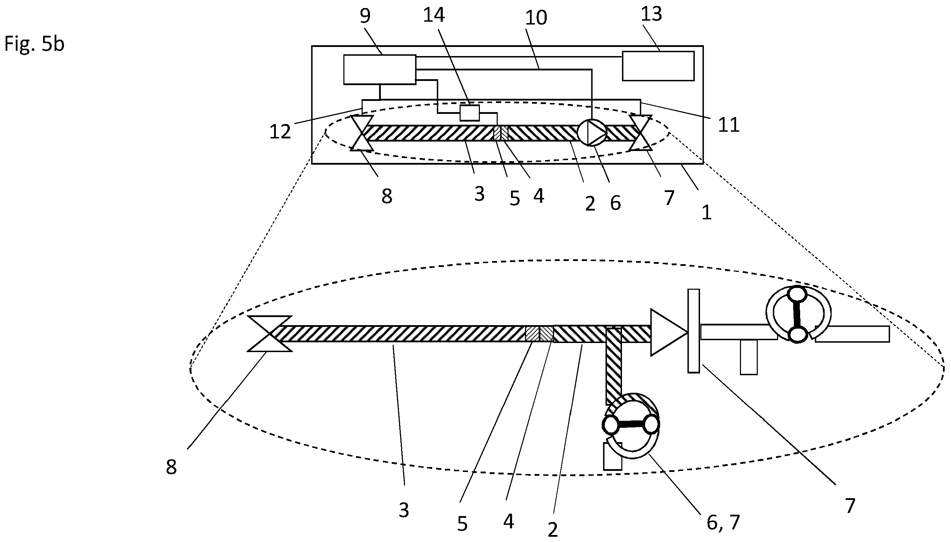

Die Figuren 5a und 5b zeigen weitere Ausführungsformen des medizinischen Geräts 1. Die Ausführungsform unterscheidet sich von den Ausführungsformen, wie zu Figs. 2, 3 beschrieben dadurch, dass die Pumpe 6 außerhalb des ersten Leitungsabschnitt 2 angeordnet ist. Das Absperrelement 8 kann ein Rückschlagventil sein, es kann aber auch ein anderes in dieser Beschreibung genanntes Absperrelement sein

Die Pumpe 6 kann eine Blutpumpe einer Blutbehandlungsmaschine sein. Die Blutpumpe kann eingerichtet sein, während einer Blutbehandlung Blut in einer Blutleitung 21 zu pumpen. Bei dem ersten Leitungsabschnitt 2 kann es sich um eine Flüssigkeitsversorgungsleitung handeln. Während einer Behandlung kann Flüssigkeit aus dem ersten Leitungsabschnitt 2 in die Blutleitung 21 überführt werden.

Durch den mittels der außerhalb angeordneten Pumpe 6 erzeugten Unterdruck kann der Leitungsabschnitt 2 bzw. zumindest ein Teilbereich davon in eine Spannungsposition gebracht werden, so dass beim Lösen der Leitungsabschnitte 2 und 3 Fluid in den zweiten Leitungsabschnitt 2 gesaugt wird.

Fig. 5b zeigt eine Variante der Ausführungsform aus Fig. 5a, bei in dem ersten Leitungsabschnitt 2 zusätzlich eine Abzweigung zu der Pumpe 6, beispielsweise eine Substituatpumpe für eine Haemodiafiltrationsbehandlung, vorgesehen ist. In dieser Ausführungsform übernimmt die Pumpe 6 auch die Funktion des Absperrelements 7 für diesen Zweig des ersten Leitungsabschnitts 2. Hierbei kann der Unterdruck in dem ersten Leitungsabschnitt 2 durch die Pumpe 6 erzeugt werden.

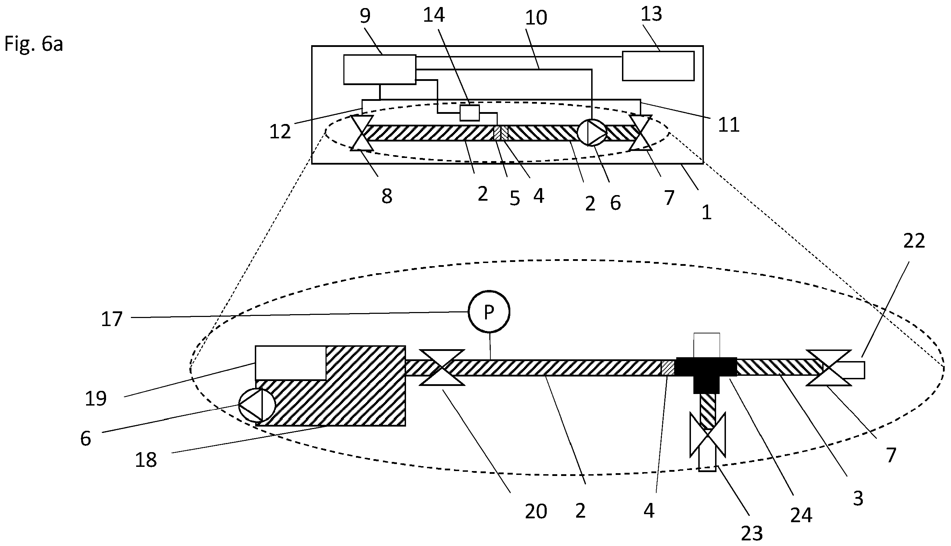

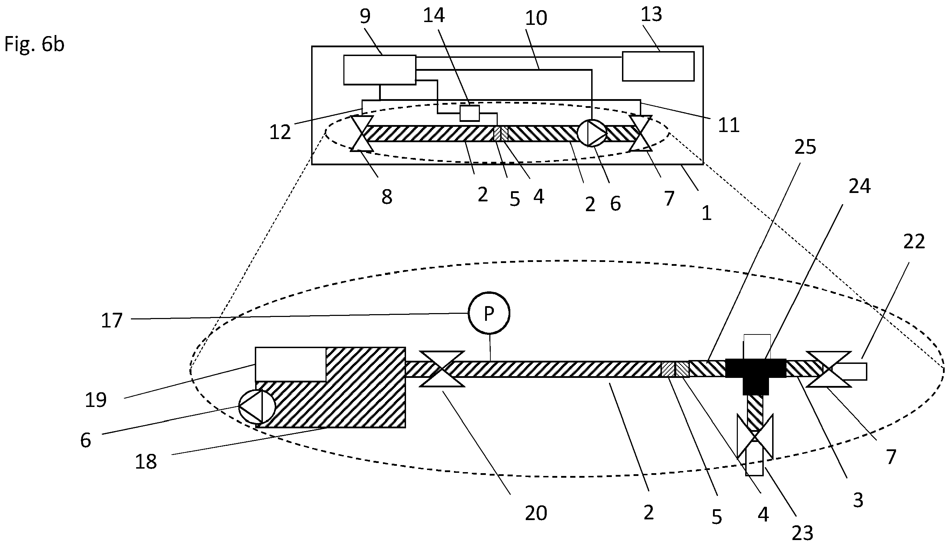

Fig. 6a und 6b zeigen Ausführungsformen des medizinischen Geräts 1 wie im Zusammenhang mit Fig. 4 beschrieben. In diesen Ausführungsform umfasst der zweite Leitungsabschnitt 3 als Disposable zumindest Teile einer arterielle 22 und einer venöse 23 Leitung, welche miteinander über ein T Stück 24 verbunden sind. Wie in Fig. 6a gezeigt, kann das T Stück 24 direkt mit dem ersten Leitungsabschnitt 2, beispielsweise über einen Abflussport einer Blutbehandlungsmaschine, verbunden sein. Bei der in Fig. 6b gezeigten Ausführungsform ist das T Stück 24 nicht direkt sondern über einen weiteren Leitungsabschnitt 25 mit dem ersten Leitungsabschnitt 2, beispielsweise einem Abflussport einer Blutbehandlungsmaschine, verbunden. Das T Stück 24 kann auch die Form eines Y Stücks haben. Der Abflussport kann auch ein sogenannter Spülport sein, mit dem Spülflüssigkeit aus dem zweiten Leitungsabschnitt 3 in den ersten Leitungsabschnitt 2 beim Spülen der arterielle 22 und/oder der venöse 23 Leitung überführt werden kann.

Die Konfiguration, wie im Zusammenhang mit den Figuren 6a uns 6b beschrieben, kann insbesondere beim Primen/Füllen des Schlauchsets / Disposables vor dem Beginn einer Behandlung verwendet werden. Bei der Diskonnektion der ersten und zweiten Leitungsabschnitte 2, 3 soll Fluid vorzugsweise in Richtung des ersten Leitungsabschnitts (zur geräteseitigen Hydraulik 18) gesaugt werden.

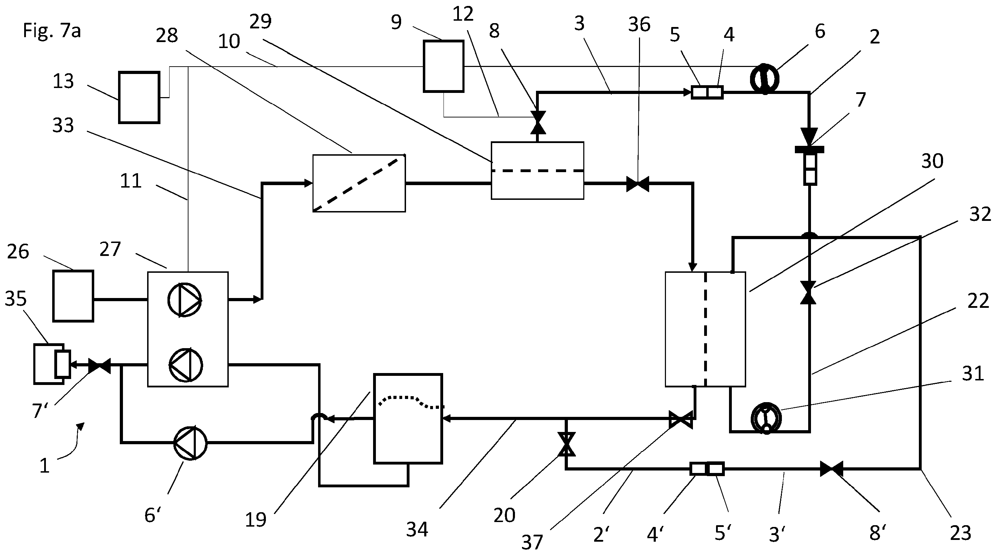

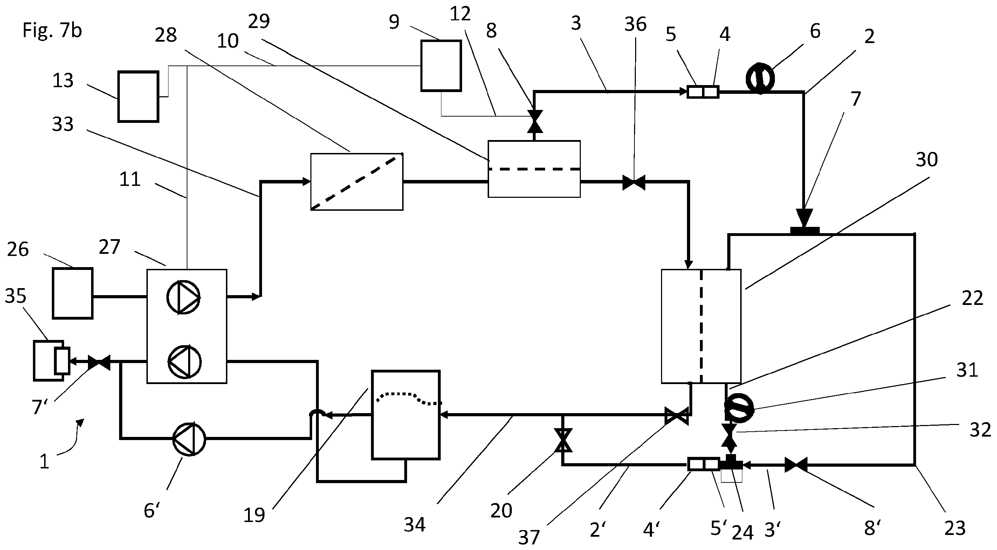

In Fig. 7a und 7b sind Ausführungsformen des medizinischen Geräts 1 in Form eines Dialysegerätes schematisch dargestellt. In den Figuren sind einige Bauteile optional, insbesondere können einige Bauteile als Disposable ausgeführt sein und müssen nicht fester Bestandteil des medizinischen Gerätes 1 sein. Die Dialysegeräte unterscheiden sich lediglich im Hinblick auf ihre Disposables bzw. der Konfiguration der Leitungsführung. In dem medizinischen Gerät 1 sind können zwei Diskonnektionsstellen (Referenzzeichen 2, 3, 4, 5 einerseits und Referenzzeichen 2', 3', 4', 5', andererseits) oder nur eine der beiden vorliegen. Die beiden Ausführungsformen sind beispielhaft. Die Diskonnektionsstelle kann eine Verbindungsstelle im Zufluss von Flüssigkeit zum extrakorporalen Blutleitungssystem sein, beispielsweise die Verbindungstelle des ersten Leitungsabschnitts 2 mit dem zweiten Leitungsabschnitt 3, oder an deren jeweiligen Enden angeordneten Konnektoren 4, 5. Die Diskonnektionsstelle kann eine Verbindungsstelle im Ablauf vom extrakorporalen Blutleitungssystem sein, beispielsweise die Verbindungstelle des ersten Leitungsabschnitts 2' mit dem zweiten Leitungsabschnitt 3', oder an deren jeweiligen Enden angeordneten Konnektoren 4', 5'. Die in dieser Beschreibung von Ausführungsformen bzw. Ausgestaltungen können auch anstelle der hier explizit beschriebenen Bauteile und Ausgestaltungen vorliegen.

Folgende Bauteile weist oder kann das Dialysegerät als medizinisches Gerät 1 aufweisen:
Flüssigkeitsquelle 26, Bilanziersystem mit Pumpe 27, erster Sterilfilter (optional) 28, zweiter Sterilfilter (optional) 29, Dialysator (optional) 30, Entlüftungskammer (optional) 19, Ultrafiltrationspumpe (optional) 6', Priming- oder Substituatport 5, Priming- oder Substituatpumpe (optional) 6, Abflussport 4', Blutpumpe 31, Steuerung 9, Benutzerschnittstelle 13, Signalleitungen (nur eine Auswahl gezeigt) 10, 11, 12, venöse Klemme 8', arterielle Klemme (optional) 32, ein Vordialysatorabsperrelement (optional) 36, ein Nachdialysatorabsperrelement (optional) 37, ein erstes Abflussleitungsabsperrelement 7' (optional), ein erstes Primingleitungabsperrelement 8, ein zweites Primingleitungabsperrelement 7, ein Abflusssperrelement (optional) 20, und T bzw. Y Stück. (optional) 24.

Die Bauteile können mit flüssigkeitsführenden Leitungen wie folgt verbunden sein: Die Flüssigkeit, in der Regel eine physiologische Flüssigkeit oder Dialysat, wird von der Flüssigkeitsquelle 26 in einer Dialysatleitung 33 durch das Bilanziersystem 27, optional durch den ersten Sterilfilter 28, zum Dialysator 30 gepumpt und dann von dem Dialysator 30 in einer Abflussleitung 34, optional durch eine Entlüftungskammer 19, wieder durch das Bilanziersystem 27 in einen Abfluss 35 (nicht Teil des medizinischen Gerätes 1) verworfen. Die Dialysatleitung 33 kann eine Abzweigungsleitung, in Form eines zweiten Leitungsabschnitts 3, aufweisen, beispielsweise optional über einen zweiten Sterilfilter 29, der über einen Priming- oder Substituatport 5 zu einer sogenannten Priming- oder Substituatleitung, in Form eines zweiten Leitungsabschnitt 2, führen kann. Diese Priming- oder Substituatleitung 2 kann mit einer arteriellen Blutleitung 22 oder einer venösen Blutleitung 23 verbunden sein. Flüssigkeit, z.B. Blut während der Behandlung oder Priming- bzw. Spülflüssigkeit in der Primingphase, in der oder den Blutleitungen 22, 23 kann mittels einer Blutpumpe 31 gepumpt werden. Das Bilanziersystem sorgt dafür, dass dem Patienten lediglich eine vorgegebene Menge an Flüssigkeit entzogen wird. Es sind verschiedene Bilanziersysteme bekannt, beispielsweise könne durch Flußmessung die Menge der Flüssigkeit, die zum Patienten gepumpt wird und die Menge, die vom Patienten weggepumpt wird bestimmt werden und das Delta - wie verschrieben - eingestellt werden, so dass eine gewünschte Ultrafiltrationsrate, in anderen Worten Nettobilanzrate, verwirklicht wird. Ein anderes Bilanziersystem ist in den Fig. 7a und 7b gezeigt. Hier wird mittels eines beispielsweise volumetrischen Bilanziersystem 26 dasselbe Volumen in Richtung des Patienten gepumpt wie vom Patienten weggepumpt wird. Eine parallel dazu geschaltete Ultrafiltrationspumpe 6' pumpt zusätzlich Flüssigkeit vom Patienten weg und erzeugt somit die Nettobilanz bzw. Ultrafiltrationsrate.

Zusätzlich kann das medizinische Gerät 1 noch eine Reihe von Absperrelemente aufweisen. Beispielsweise kann das das Gerät 1 ein venöse Absperrelement 8' (venöse Klemme), eine arterielles Absperrelement 32 (arterielle Klemme), ein Vordialysatorabsperrelement 36, ein Nachdialysatorabsperrelement 37, ein erstes Abflussleitungsabsperrelement 7', ein erstes und ein zweites Primingleitungabsperrelement aufweisen 7, 8.

Der Unterschied der in den Figs. 7a und 7b dargestellten Ausführungsformen besteht darin, dass bei der in Fig. 7a dargestellten Ausführungsform die Priming- oder Substituatleitung 2 mit einem Ende der der arteriellen Blutleitung 22 verbunden ist - normalerweise das Ende, das während der Behandlung mit dem Patienten verbunden ist, und nur die venöse Blutleitung 23 mit einem - normalerweise das patientenseitige - Ende mit dem Abflussport 4' verbunden ist. In der Fig. 7b dargestellten Ausführungsform ist die Priming- oder Substituatleitung 2 mit einem entlang der venösen Blutleitung 23 angeordneten Port verbunden und auch das - normalerweise das patientenseitige - Ende der arteriellen Blutleitung 22 ist mit dem Drain- oder Rinseport 4' verbunden. In einer weiteren Ausführungsform ist die Priming- oder Substituatleitung mit einem entlang der arteriellen Blutleitung angeordneten Port verbunden.

Insbesondere folgende Bauteile bzw. Leitungen können als Disposable ausgeführt sein: der Dialysator 30, die arterielle Blutleitung 22, die venöse Blutleitung 23, die Priming- oder Substituatleitung 2. Diese Leitung zusammen können ein Schlauchset oder eine Kassettensystem bilden. Ein Kassettensystem bedeutet, dass wenigstens zwei dieser Leitungen unlösbar miteinander verbunden sind und/oder die Leitungen wenigsten teilweise durch flexible Schläuche, sondern durch formstabilen Kanäle gebildet werden.

Das medizinische Gerät 1 kann beispielsweise eingerichtet sein, um vor der Behandlung das Schlauchset oder das Kassettensystem mit physiologischer Flüssigkeit zu füllen. Dazu kann beispielsweise die Steuerung 9 programmiert sein, beispielsweise in einem Füllmodus, der auch Primingmodus genannt werden kann, mittels der Pumpen des Bilanziersystems 27 aus der Flüssigkeitsquelle 26 Flüssigkeit über den Priming- oder Substituatport 4 in das Schlauchset oder das Kassettensystem zu überführen. In einem weiteren Verfahrensschritt, beispielsweise einem Spülmodus, nach dem Füllen, kann das Schlauchset oder das Kassettensystem gespült werden, wobei Flüssigkeit durch das Schlauchset oder das Kassettensystem gespült wird und durch den Abflussport 4' in die Abflussleitung 34 gespült wird. Zur Behandlung muss die venösen Leitung 23 mit dem Patienten verbunden werden. Hierzu wird, beispielsweise in der Ausführungsform der Fig. 7a, das Ende der venösen Leitung, das mit dem optionalen Übergangsstück 5' mit dem Abflussport 4' verbunden ist, vom Abflussport 4' gelöst. Bevor jedoch dieses Lösen erfolgt, das manuell oder automatisch erfolgen kann, veranlasst die Steuerung 9, dass zumindest ein erster Leitungsabschnitt 2' der mit dem Abflussport 4' verbunden ist und ein Teil der venösen Leitung als zweiter Leitungsabschnitt 3' zumindest auf der Seite der venösen Leitung 23 abgeschlossen wird. Die Steuerung kann beispielsweise die Ultrafiltrationspumpe 6' ansteuern und mit dieser Flüssigkeit abpumpen, so dass ein Unterdruck entsteht. Auf Grund der elastischen Eigenschaft des ersten Leitungsabschnitts 2' verformt sich zumindest ein Teil des ersten Leitungsabschnitts 2' in eine Spannungsposition. Der erste Leitungsabschnitts 2' kann auch elastischer sein als der zweite Leitungsabschnitt 3'. Dann kann die Steuerung wenigstens eines, optional mehrere Absperrelemente 7', ansteuern, um das System in dieser Spannungsposition zu halten. Bei einem, optional manuell oder automatischen, anschließende Lösen des zweiten Leitungsabschnitt 3' vom Drain- oder Rinseport 4', kann des erste Leitungsabschnitt 2' relaxieren.

An dieser Stelle sei darauf hingewiesen, dass die medizinischen Geräte 1, wie im Zusammenhang mit den Figuren 7a und 7b beschrieben, ferner über eines oder mehrere der folgenden Bauteile mit der in dieser Beschreibung beschriebenen Funktion aufweisen kann: einen Gasreservoir 19, ein Ventil 20 zur Vergrößerung der elastischen Eigenschaft des ersten Leitungsabschnitt 2', eine Drucksensor (nicht gezeigt).

Das medizinische Gerät 1 kann ferner für einen Diskonnektionsschritt nach der Behandlung eingerichtet sein. Dazu kann die Steuerung 9 das Absperrelement 8 schließen und die Pumpe 6 betreiben. Die Pumpe 6 kann eine peristaltische Pumpe in Form einer Substituatpumpe sein. Alternativ kann auch die Blutpumpe 31 genutzt werden, um den Unterdruck in dem Leitungsabschnitt 2 zu generieren bzw. Flüssigkeit aus diesem abzupumpen. Das Absperrelement 7 kann ein Rückschlagventil sein und kann auch Teil eines Übergangsstücks oder Teil des arteriellen Schlauches 22 sein, so dass ein aktives Schließen dieses Absperrelements nicht erforderlich sein muss. Bei anderen Ausgestaltungen des Absperrelements 7 kann die Steuerung programmiert sein, das Absperrelement 7 nach Betreiben der Pumpe 6 zu schließen. Auf Grund der elastischen Eigenschaft des ersten Leitungsabschnitts 2 verformt sich zumindest ein Teil des ersten Leitungsabschnitts 2 in eine Spannungsposition. Der erste Leitungsabschnitts 2 kann auch elastischer sein als der zweite Leitungsabschnitt 3. Bei einem, optional manuell oder automatischen, anschließende Lösen des zweiten Leitungsabschnitt 3 vom Priming- oder Substituatport 5 kann des erste Leitungsabschnitt 2 relaxieren.

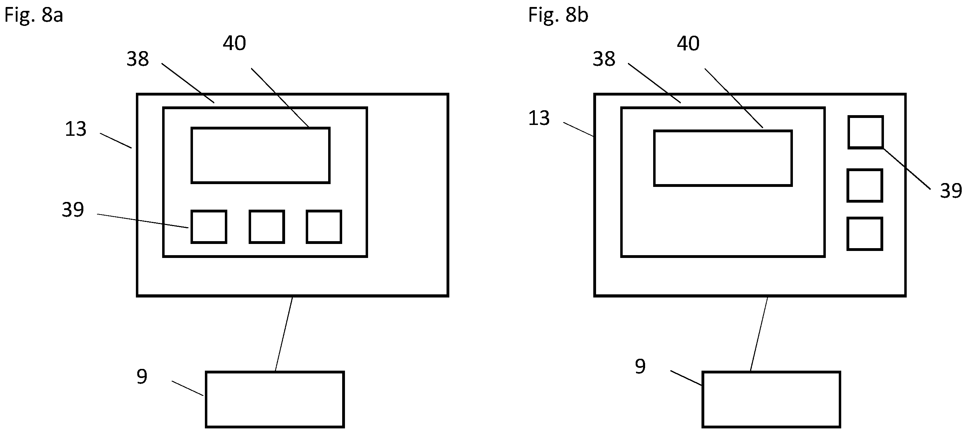

Die Figuren 8a und 8b zeigen schematisch die Benutzerschnittstelle 13. Die Benutzerschnittstelle 13 kann einen Bildschirm 38 und wenigstens einen Knopf 39 aufweisen. Der Bildschirm 38 kann ein Touchscreen sein und der Knopf 39 kann als Softkey, ein auf dem Touchscreen zu betätigender Knopf, ausgeführt sein, wie in Fig. 7a dargestellt. Der Knopf 39 kann auch als Hardkey, ein separat vom Bildschirm vorgesehener Knopf, ausgeführt sein, wie in Fig. 7b dargestellt. Die Steuerung 9 kann eingerichtet sein über eine Datenleitung Instruktionen an die Benutzerschnittstelle 13 zu senden oder von dieser solche zu empfangen. Beispielsweise kann die Benutzerschnittstelle 1 programmiert sein nach Betätigen des Knopfs 39, die Steuerung zu veranlassen, in den Diskonnektionsmodus zu wechseln bzw. den Diskonnektionsmodus zu starten. Die Steuerung 9 kann programmiert sein, eine Sequenz von Verfahren durchzuführen und eine Nachricht 40, beispielsweise zur Anzeige, an die Benutzerschnittstelle 13 zu schicken, wenn eine oder mehrere oder alle der folgenden Situationen eintreten bzw. die Steuerung in der Abarbeitung eines Programms an diese Punkt gelangt: der Programmablauf erlaubt eine Aktivierung des Diskonnektionsmodus, der Diskonnektionsmodus kann gestartet werden, ein Lösen der Verbindung kann erfolgen, nachdem die maschinenseitig durchzuführenden Verfahrensschritte erfolgt sind, ein Desinfektionsverfahren muss durchgeführt werden, beispielsweise weil die Steuerung erkannt hat, dass eine Behandlung vorbereitet oder durchgeführt werden soll oder ein Sensor, beispielsweise ein Pin, der die Anwesenheit eines Disposables anzeigt, der Steuerung anzeigt, dass ein Disposable von der Maschine entfernt wurde, ohne das die die maschinenseitig durchzuführenden Verfahrensschritte erfolgt sind.

Wenn hierin von einer Ausführungsform die Rede ist, so ist hierunter eine erfindungsgemäße, rein exemplarische Ausführungsform zu verstehen.

Erfindungsgemäße Ausführungsformen können eines oder mehrere der oben genannten Merkmale in beliebiger Kombination aufweisen, sofern für den Fachmann die konkrete Ausführungsform nicht als technisch unmöglich erkennbar ist.

## Patentansprüche

1. Verfahren zur Diskonnektion zweier fluidführender Leitungsabschnitte eines medizinischen Geräts, insbesondere eines medizinischen Geräts nach einem der Ansprüche 7 bis 17, welche miteinander lösbar verbunden sind, mit den Schritten:
Einschließen eines Fluidvolumens in den zwei Leitungsabschnitten,
Erzeugen eines Unterdrucks in den zwei Leitungsabschnitten, und
Lösen der Verbindung der Leitungsabschnitte,
**dadurch gekennzeichnet, dass**
durch das Erzeugen eines Unterdrucks in den zwei Leitungsabschnitten eine elastische Verformung im und/oder am ersten Leitungsabschnitt, welcher zumindest teilweise eine elastische Eigenschaft aufweist, aus einer Ausgangsposition in eine Spannungsposition erfolgt, wobei ein vom ersten Leitungsabschnitt umfasstes Fluidvolumen in der Spannungsposition kleiner ist als ein in der Ausgangsposition umfasstes Fluidvolumen, und
sich bei dem Lösen der Verbindung der Leitungsabschnitte das vom ersten Leitungsabschnitt umfasste Fluidvolumen der Spannungsposition vergrößert.

2. Verfahren nach Anspruch 1, wobei der erste Leitungsabschnitt zumindest einen Teilbereich aufweist, welcher relativ zu dem zweiten Leitungsabschnitt elastischer ist.

3. Verfahren nach Anspruch 1 oder 2, wobei der erste Leitungsabschnitt und der zweite Leitungsabschnitt zumindest einen Teilbereich aufweisen, welcher relativ zu dem Rest des ersten Leitungsabschnitts und des zweiten Leitungsabschnitts elastischer sind.

4. Verfahren nach einem der vorhergehenden Ansprüche, dass die elastische Eigenschaft gegeben ist durch ein elastisches Material und/oder eine Geometrie mit elastischer Rückstellkraft und/oder eingeschlossenes Gas.

5. Verfahren nach einem der vorhergehenden Ansprüche, wobei der erste Leitungsabschnitt Teil eines gerätseitigen Fluidiksystems, insbesondere des medizinischen Geräts, ist und der zweite Leitungsabschnitt Teil eines Disposables ist, oder der zweite Leitungsabschnitt Teil eines gerätseitigen Fluidiksystems, insbesondere des medizinischen Geräts, ist und der erste Leitungsabschnitt Teil eines Disposables ist.

6. Verfahren nach einem der vorhergehenden Ansprüche, wobei sich in dem ersten und zweiten Leitungsabschnitt weitestgehend ein physiologische Fluid und kein Blut befindet.

7. Medizinisches Gerät (1), mit mindestens zwei voneinander lösbaren fluidführenden Leitungsabschnitten (2,3), mit
wenigstens einem ersten und einem zweiten Absperrelement (7,8) zum Einschließen eines Fluidvolumens in den zwei Leitungsabschnitten (2,3),
einer Pumpe (6) zum Erzeugen eines Unterdrucks in den zwei Leitungsabschnitten (2,3), und
einer Steuerung (9) zum Ansteuern der Pumpe (6),
**dadurch gekennzeichnet, dass**
ein erster Leitungsabschnitt der zwei fluidführenden Leitungsabschnitte (2,3) zumindest teilweise eine elastische Eigenschaft aufweist;
und
die Steuerung (9) programmiert ist, in einem Diskonnektionsmodus die Pumpe (6) zum Erzeugen des Unterdrucks zu betreiben, so dass durch das Erzeugen eines Unterdrucks in den zwei Leitungsabschnitten (2,3) eine elastische Verformung im und/oder am ersten Leitungsabschnitt aus einer Ausgangsposition in eine Spannungsposition erfolgt.

8. Medizinisches Gerät (1) nach Anspruch 7, wobei die Steuerung programmiert ist vor Erzeugung des Unterdrucks eines der Absperrelemente (7,8) zu schließen, zum einseitigen Abschließen des Fluidvolumens und / oder nach Erzeugung des Unterdrucks eines der Absperrelemente (7,8) zum Einschließen des Fluidvolumens zu schließen.

9. Medizinisches Gerät (1) nach Anspruch 7 oder 8, wobei der erste oder zweite Leitungsabschnitt (2,3) Teil eines gerätseitigen Fluidiksystems des medizinischen Geräts (1) ist.

10. Medizinisches Gerät (1) nach Anspruch 9, aufweisend
eine Fluidquelle (26), insbesondere eine Fluidquelle für eine physiologische Flüssigkeit, fluidisch verbunden mit dem zweiten Leitungsabschnitt (3),
optional einen Sterilfilter (28) fluidisch angeordnet zwischen und verbunden mit der Fluidquelle (26) und dem zweiten Leitungsabschnitt (3), und
einem medizingeräteseitigen Konnektor (5) an einem Ende des zweiten Leitungsabschnitts (3) zum Verbinden mit einem Ende des ersten Leitungsabschnitts (2).

11. Medizinisches Gerät (1) nach Anspruch 9, aufweisend
eine Abflussleitung (34), wobei der erste Leitungsabschnitt (2) mit der Abflussleitung fluidisch verbunden ist oder einen Teil dieser ist,
einem medizingeräteseitigen Konnektor (4) an einem Ende des ersten Leitungsabschnitts (2) zum Verbinden mit einem Ende des zweiten Leitungsabschnitts (3).

12. Medizinisches Gerät (1) nach einem der Ansprüche 7 bis 11, wobei die Pumpe (6) eine peristaltische Pumpe ist und wenigstens ein Aktor der peristaltischen Pumpe Teil des ersten oder zweiten Absperrelements (7, 8) ist oder das erste oder zweite Absperrelement (7,8) ist.

13. Medizinisches Gerät (1) nach einem der Ansprüche 7 bis 12, wobei die Pumpe (6) eine Ultrafiltrationspumpe und / oder einer Blutpumpe und / oder einer Substituatpumpe eines extrakorporalen Blutbehandlungsgerätes, insbesondere eines Dialysegerätes, ist.

14. Medizinisches Gerät (1) nach einem der Ansprüche 7 bis 13, aufweisend
eine Benutzerschnittstelle (13) zum Eingeben einer Anweisung durch einen Nutzer, wobei die Steuerung (9) programmiert ist, den Diskonnektionsmodus zu aktivieren oder die Pumpe (6) zu starten als Reaktion auf eine Eingabe der Anweisung an der Benutzerschnittstelle (13), und/oder
wobei die Steuerung (9) programmiert ist, eine Mehrzahl von Modi zu aktivieren und einen Wechsel aus einem der Modi in den Diskonnektionsmodus automatisch durchzuführen.

15. Medizinisches Gerät (1) nach einem der Ansprüche 9 bis 14, mit
dem jeweils anderen Leitungsabschnitt der zwei Leitungsabschnitte (2,3), wobei der erste Leitungsabschnitt (2) zumindest teilweise eine elastische Eigenschaft aufweist, wobei ein vom ersten Leitungsabschnitt (2) umfasstes Fluidvolumen in der Spannungsposition kleiner ist als ein in der Ausgangsposition umfasstes Fluidvolumen, und
wobei optional der jeweils andere Leitungsabschnitt der zwei Leitungsabschnitte (2,3) Teil eines Disposables ist, insbesondere eines im Rahmen einer Blutbehandlung zum Einsatz kommenden Schlauchsets oder Kassettensystems, oder optional beide Leitungsabschnitte (2,3) Teil eines oder mehrerer Disposables sind, insbesondere eines im Rahmen einer Blutbehandlung zum Einsatz kommenden Schlauchsets oder Kassettensystems.

16. Medizinisches Geräte (1) nach Anspruch 15, wobei der erste Leitungsabschnitt (2) zumindest einen Teilbereich aufweist, welcher relativ zu dem zweiten Leitungsabschnitt (3) elastischer ist.

17. Medizinisches Geräte (1) nach Anspruch 15 oder 16, wobei der erste Leitungsabschnitt (2) und der zweite Leitungsabschnitt (3) zumindest einen Teilbereich aufweisen, welcher relativ zu dem Rest des ersten Leitungsabschnitts (2) und des zweiten Leitungsabschnitts (3) elastischer sind.

## Claims

1. A method for disconnecting two fluid-conducting line sections of a medical device, in particular of a medical device according to one of claims 7 to 17, which are releasably connected to one another, comprising the steps of:
enclosing a fluid volume in the two line sections, generating a negative pressure in the two line sections, and releasing the connection of the line sections,
**characterized in that**
by generating a negative pressure in the two line sections, an elastic deformation in and/or on the first line section, which has at least partially an elastic property, takes place from an initial position into a tension position, wherein a fluid volume encompassed by the first line section in the tension position is smaller than a fluid volume encompassed in the initial position, and upon releasing the connection of the line sections, the fluid volume encompassed by the first line section of the tension position increases.

2. The method according to claim 1, wherein the first line section has at least one partial region which is more elastic relative to the second line section.

3. The method according to claim 1 or 2, wherein the first line section and the second line section have at least one partial region which is more elastic relative to the rest of the first line section and the second line section.

4. The method according to one of the preceding claims, wherein the elastic property is provided by an elastic material and/or a geometry with elastic restoring force and/or enclosed gas.

5. The method according to one of the preceding claims, wherein the first line section is part of a device-side fluidic system, in particular of the medical device, and the second line section is part of a disposable, or the second line section is part of a device-side fluidic system, in particular of the medical device, and the first line section is part of a disposable.

6. The method according to one of the preceding claims, wherein a physiological fluid and no blood is located substantially in the first and second line section.

7. A medical device (1), comprising at least two fluid-conducting line sections (2,3) releasable from one another, comprising
at least one first and one second shut-off element (7,8) for enclosing a fluid volume in the two line sections (2,3),
a pump (6) for generating a negative pressure in the two line sections (2,3), and a control unit (9) for actuating the pump (6),
**characterized in that**
a first line section of the two fluid-conducting line sections (2,3) has at least partially an elastic property;
and the control unit (9) is programmed to operate the pump (6) in a disconnection mode for generating the negative pressure, so that by generating a negative pressure in the two line sections (2,3), an elastic deformation in and/or on the first line section takes place from an initial position into a tension position.

8. The medical device (1) according to claim 7, wherein the control unit is programmed, before generation of the negative pressure, to close one of the shut-off elements (7,8) for one-sidedly closing off the fluid volume and/or, after generation of the negative pressure, to close one of the shut-off elements (7,8) for enclosing the fluid volume.

9. The medical device (1) according to claim 7 or 8, wherein the first or second line section (2,3) is part of a device-side fluidic system of the medical device (1).

10. The medical device (1) according to claim 9, comprising
a fluid source (26), in particular a fluid source for a physiological liquid, fluidically connected to the second line section (3), optionally a sterile filter (28) fluidically arranged between and connected to the fluid source (26) and the second line section (3), and
a medical-device-side connector (5) at one end of the second line section (3) for connecting to one end of the first line section (2).

11. The medical device (1) according to claim 9, comprising
a discharge line (34), wherein the first line section (2) is fluidically connected to the discharge line or is a part thereof,
a medical-device-side connector (4) at one end of the first line section (2) for connecting to one end of the second line section (3).

12. The medical device (1) according to one of claims 7 to 11, wherein the pump (6) is a peristaltic pump and at least one actuator of the peristaltic pump is part of the first or second shut-off element (7, 8) or is the first or second shut-off element (7,8).

13. The medical device (1) according to one of claims 7 to 12, wherein the pump (6) is an ultrafiltration pump and/or a blood pump and/or a substitute fluid pump of an extracorporeal blood treatment apparatus, in particular a dialysis apparatus.

14. The medical device (1) according to one of claims 7 to 13, comprising a user interface (13) for inputting an instruction by a user, wherein the control unit (9) is programmed to activate the disconnection mode or to start the pump (6) in response to an input of the instruction at the user interface (13),
and/or
wherein the control unit (9) is programmed to activate a plurality of modes and to automatically perform a change from one of the modes into the disconnection mode.

15. The medical device (1) according to one of claims 9 to 14, with the respective other line section of the two line sections (2,3), wherein the first line section (2) has at least partially an elastic property, wherein a fluid volume encompassed by the first line section (2) in the tension position is smaller than a fluid volume encompassed in the initial position, and
wherein optionally the respective other line section of the two line sections (2,3) is part of a disposable, in particular of a tube set or cassette system used in the context of a blood treatment, or optionally both line sections (2,3) are part of one or more disposables, in particular of a tube set or cassette system used in the context of a blood treatment.

16. The medical device (1) according to claim 15, wherein the first line section (2) has at least one partial region which is more elastic relative to the second line section (3).

17. The medical device (1) according to claim 15 or 16, wherein the first line section (2) and the second line section (3) have at least one partial region which is more elastic relative to the rest of the first line section (2) and the second line section (3).

## Revendications

1. Procédé de déconnexion de deux sections de conduite véhiculant un fluide d'un appareil médical, en particulier d'un appareil médical selon l'une des revendications 7 à 17, lesquelles sont reliées l'une à l'autre de manière détachable, comprenant les étapes consistant à :
enfermer un volume de fluide dans les deux sections de conduite, générer une dépression dans les deux sections de conduite, et desserrer la liaison des sections de conduite,
**caractérisé en ce que**
par la génération d'une dépression dans les deux sections de conduite, une déformation élastique dans et/ou sur la première section de conduite, laquelle présente au moins partiellement une propriété élastique, s'effectue d'une position initiale vers une position de tension, un volume de fluide englobé par la première section de conduite dans la position de tension étant inférieur à un volume de fluide englobé dans la position initiale, et
lors du desserrage de la liaison des sections de conduite, le volume de fluide englobé par la première section de conduite de la position de tension augmente.

2. Procédé selon la revendication 1, dans lequel la première section de conduite présente au moins une zone partielle qui est plus élastique relativement à la deuxième section de conduite.

3. Procédé selon la revendication 1 ou 2, dans lequel la première section de conduite et la deuxième section de conduite présentent au moins une zone partielle qui est plus élastique relativement au reste de la première section de conduite et de la deuxième section de conduite.

4. Procédé selon l'une des revendications précédentes, dans lequel la propriété élastique est donnée par un matériau élastique et/ou une géométrie présentant une force de rappel élastique et/ou un gaz enfermé.

5. Procédé selon l'une des revendications précédentes, dans lequel la première section de conduite fait partie d'un système fluidique côté appareil, en particulier de l'appareil médical, et la deuxième section de conduite fait partie d'un consommable, ou la deuxième section de conduite fait partie d'un système fluidique côté appareil, en particulier de l'appareil médical, et la première section de conduite fait partie d'un consommable.

6. Procédé selon l'une des revendications précédentes, dans lequel un fluide physiologique et pas de sang se trouve pour l'essentiel dans la première et la deuxième section de conduite.

7. Appareil médical (1), comprenant au moins deux sections de conduite véhiculant un fluide (2,3), détachables l'une de l'autre, comprenant
au moins un premier et un deuxième élément d'obturation (7,8) pour enfermer un volume de fluide dans les deux sections de conduite (2,3),
une pompe (6) pour générer une dépression dans les deux sections de conduite (2,3), et
une commande (9) pour piloter la pompe (6),
**caractérisé en ce que**
une première section de conduite des deux sections de conduite véhiculant un fluide (2,3) présente au moins partiellement une propriété élastique ;
et la commande (9) est programmée, dans un mode de déconnexion, pour faire fonctionner la pompe (6) afin de générer la dépression, de sorte que, par la génération d'une dépression dans les deux sections de conduite (2,3), une déformation élastique dans et/ou sur la première section de conduite s'effectue d'une position initiale vers une position de tension.

8. Appareil médical (1) selon la revendication 7, dans lequel la commande est programmée, avant la génération de la dépression, pour fermer l'un des éléments d'obturation (7,8), afin de fermer unilatéralement le volume de fluide, et/ou, après la génération de la dépression, pour fermer l'un des éléments d'obturation (7,8) afin d'enfermer le volume de fluide.

9. Appareil médical (1) selon la revendication 7 ou 8, dans lequel la première ou la deuxième section de conduite (2,3) fait partie d'un système fluidique côté appareil de l'appareil médical (1).

10. Appareil médical (1) selon la revendication 9, comprenant
une source de fluide (26), en particulier une source de fluide pour un liquide physiologique, reliée fluidiquement à la deuxième section de conduite (3), un filtre stérile (28) optionnellement disposé fluidiquement entre la source de fluide (26) et la deuxième section de conduite (3) et relié à celles-ci, et
un connecteur côté appareil médical (5) à une extrémité de la deuxième section de conduite (3) pour relier à une extrémité de la première section de conduite (2).

11. Appareil médical (1) selon la revendication 9, comprenant
une conduite d'évacuation (34), la première section de conduite (2) étant reliée fluidiquement à la conduite d'évacuation ou en constituant une partie,
un connecteur côté appareil médical (4) à une extrémité de la première section de conduite (2) pour relier à une extrémité de la deuxième section de conduite (3).

12. Appareil médical (1) selon l'une des revendications 7 à 11, dans lequel la pompe (6) est une pompe péristaltique et au moins un actionneur de la pompe péristaltique fait partie du premier ou du deuxième élément d'obturation (7, 8) ou constitue le premier ou le deuxième élément d'obturation (7,8).

13. Appareil médical (1) selon l'une des revendications 7 à 12, dans lequel la pompe (6) est une pompe d'ultrafiltration et/ou une pompe à sang et/ou une pompe de substituat d'un appareil de traitement extracorporel du sang, en particulier d'un appareil de dialyse.

14. Appareil médical (1) selon l'une des revendications 7 à 13, comprenant une interface utilisateur (13) pour la saisie d'une instruction par un utilisateur, la commande (9) étant programmée pour activer le mode de déconnexion ou pour démarrer la pompe (6) en réponse à une saisie de l'instruction sur l'interface utilisateur (13),
et/ou
la commande (9) étant programmée pour activer une pluralité de modes et pour effectuer automatiquement un changement depuis l'un des modes vers le mode de déconnexion.

15. Appareil médical (1) selon l'une des revendications 9 à 14, avec l'autre section de conduite respective des deux sections de conduite (2,3), la première section de conduite (2) présentant au moins partiellement une propriété élastique, un volume de fluide englobé par la première section de conduite (2) dans la position de tension étant inférieur à un volume de fluide englobé dans la position initiale, et
dans lequel, optionnellement, l'autre section de conduite respective des deux sections de conduite (2,3) fait partie d'un consommable, en particulier d'un jeu de tuyaux ou d'un système à cassette utilisé dans le cadre d'un traitement du sang, ou, optionnellement, les deux sections de conduite (2,3) font partie d'un ou de plusieurs consommables, en particulier d'un jeu de tuyaux ou d'un système à cassette utilisé dans le cadre d'un traitement du sang.

16. Appareil médical (1) selon la revendication 15, dans lequel la première section de conduite (2) présente au moins une zone partielle qui est plus élastique relativement à la deuxième section de conduite (3).

17. Appareil médical (1) selon la revendication 15 ou 16, dans lequel la première section de conduite (2) et la deuxième section de conduite (3) présentent au moins une zone partielle qui est plus élastique relativement au reste de la première section de conduite (2) et de la deuxième section de conduite (3).
